# EUROPEAN PATENT SPECIFICATION

(11) **EP 3 986 502 B1**
(45) Date of publication and mention of the grant of the patent: **16.10.2024**
(21) Application number: 20740389.0
(22) Date of filing: 17.06.2020
(51) Int. Cl.: A61M 1/36

(54) **IMPLANTABLE VASCULAR ACCESS DEVICE**
IMPLANTIERBARE GEFÄSSZUGANGSVORRICHTUNG
DISPOSITIF D'ACCÈS VASCULAIRE IMPLANTABLE

(30) Priority: 18.06.2019 IT 201900009318; 05.06.2020 IT 202000013321
(43) Date of publication of application: 27.04.2022
(73) Proprietor: Emodial S.r.l., 44100 Cassana (Ferrara) (IT)
(72) Inventor: PECORARI, Gianni, 41037 Mirandola (MO) (IT)
(74) Representative: Dall'Olio, Christian
(86) International application number: PCT/IB2020/055640
(87) International publication number: WO 2020/254976

(56) References cited:
- US-A- 4 822 341
- US-A1- 2010 318 016
- US-A1- 2011 213 309

## Description

### FIELD OF THE INVENTION

The invention relates to the field of haemodialysis devices. In particular, the invention relates to a vascular access device for haemodialysis permanently implantable in a patient having a pre-existing arteriovenous fistula. Herein also disclosed but not part of the invention is a sterile package containing a single-use device of collecting and/or injecting blood from and to the patient utilisable in combination with a vascular access device for haemodialysis with which a collection and/or injection system for haemodialysis can be obtained which comprises both devices and which advantageously enables carrying out the surgical and haemodialysis methods described herein but which are also not part of the invention. A collection and/or injection system is, however, part of the invention.

### DESCRIPTION OF THE PRIOR ART

Haemodialysis is a treatment that enables, by means of extracorporeal circulation of blood, purifying the blood in a special haemodialysis apparatus which comprises a semi-permeable membrane through which only (or at least the majority of) the toxic substances that are to be removed pass. Patients suffering from severe renal failure are subject to this treatment. Obviously, in order to carry out the haemodialysis it is necessary to create a vascular access, i.e. two points: one from which the blood to be purified is to be collected, and the other from which the filtered blood is re-injected into the circulatory system. The vascular access most generally used is the arteriovenous fistula (also known as a FAV) which is realised by a surgical operation, creating a permanent connection between a vein and an artery which involves the widening of the portion of vein of the arteriovenous fistula as the arterial pressure is much higher than the venous pressure. Therefore the two above-mentioned points of the vascular access can be predisposed in the arteriovenous fistula, as this enables passage of the relatively high blood flows, and reduces the time necessary for the haemodialysis. Notwithstanding this, haemodialysis is a long treatment, and in fact requires from three to four hours and must be carried out two or three times a week. Consequently the arteriovenous fistula is periodically and frequently subjected to the traumas deriving from the insertion of metal needles, known as fistula needles, which are made of metal, have a diameter in the order of 1.6 mm, as well as a sharp and cutting tip. The needles are to be inserted in the arteriovenous fistula and can create traumas to the portion of wall of the FAV opposite the inlet portion, in particular because in order to insert in the vein the fistula needle is inserted with a first inclination with respect to the skin and to insert it in the FAV it is necessary to change that inclination and position the needle parallel to the fistula. This causes stress, pain and traumas to the patient (the bruising alone is testimony to this), the risk of the FAV passing completely from side to side, complications for individuals who suffer from vein fragility and a high possibility of infection. In fact, even when administering local anaesthesia, the patient, once the effect of the anaesthesia has faded, still feels pain. To obviate this, the "Buttonhole" method has been developed, which includes inserting needles in the FAV always at the same two points (collection and return) with the same angulation and depth with the purpose of creating a sort of subcutaneous channel of scar tissue that extends from the cutaneous surface to the FAV through which the needles are inserted prior to every haemodialysis session. Special devices have also been created which are to be inserted into the channel from session to session in order to keep it open until the channel has been stably formed. It is clear that it is of fundamental importance to guarantee the same angulation and the same depth during each insertion of the needle. On this matter, first the above-mentioned fistula needles (sharp and cutting) have to be used for a certain number of dialysis sessions, and only after this can needles be used that are suitable for the Buttonhole method, which needles have the same diameter as the fistula and a bevelled tip which does not cut. Therefore highly specialised personnel is necessary for practising the method, especially in the early days of treatment. Further, the Buttonhole method is correlated to a greater incidence of infections with respect to the use of cutting needles and further it is not recommended in a case in which the subcutaneous tissue is too much or too little and where there is scarred skin. Recently vascular access devices for haemodialysis have been developed which are permanently implantable in a patient having an arteriovenous fistula. These devices have a fixing surface destined to be fixed to the outer wall of the FAV proximal to the skin and which comprise a funnelling channel opening into the thinnest part in the fixing surface and in which, once implanted, the device remains stuck in the muscle. With these devices too, it is necessary to use a fistula needle which is inserted into the skin and the muscle, seeking to centre the funnelling channel and gradually, as it is being inserted, passes through the wall of the FAV at the fixing surface of the device. This device does not do away with the risk of crossing the vein (V) with the fistula needle from side to side: further, owing to the funnelling channel, there is no possibility of a variety of inclinations of the fistula needle which therefore cannot be inserted deeply, nor does it enable a reduction of the treatment time which is limited by the section of the fistula needle, alike to the Buttonhole method.

Documents US 2010/318016 A1 and US 4.822.341 describe further vascular access devices for haemodialysis permanently implantable in a patient having a pre-existing arteriovenous fistula. The device according to US 4.822.341 is implanted in series to a blood vessel. Once implanted, the device has an inlet hole and an outlet hole, respectively for the collection and re-injection of blood. This device includes a sliding valve for closing and opening the holes by sliding longitudinally with respect to the blood vessel, and once the device has been implanted the sliding valve is arranged subcutaneously. It is clear that in order to open and close the holes, the operator must cause the sliding valve to slide internally of the patient's body to oppose the action of the tissues at the valve, and that the operator also has to use a fistula needle, inserting it into the skin and the muscle, seeking to centre the holes, once open. Owing to the objective difficulties in sliding the implanted valve in the body, the device has virtually never had a practical application. Therefore a need emerges to carry out the haemodialysis sessions while limiting the stress, pain, and traumas for the patient, the damage to the FAV, the possibility of infections of the vascular access sites of the FAV, the need for highly specialised personnel and the related costs. There additionally emerges a need to reduce the number of operations the patient has to be subjected to in order to prepare her or him for haemodialysis with the aim of reducing the risks correlated with the operations and the disturbance and pain caused to the patient. Further, there is an acknowledged need to reduce the costs and/or times for haemodialysis, as well as the need for specialised healthcare operators.

### SUMMARY OF THE INVENTION

The aim of the present invention is to obviate or limit the drawbacks correlated to haemodialysis using FAV. In particular, the main aim of the present invention is to provide a haemodialysis treatment while limiting the stress, pain, and traumas for the patient, the damage to th FAV or other blood vessels, the possibility of infections of the vascular access sites of the FAV, the need for highly specialised personnel and the related costs. Additionally, a further aim of the present invention is to realise a vascular access device which is constructionally simple and economical, as well as reliable in use. A further aim of the present invention is to enable a reduction of haemodialysis times. The above-mentioned aims are attained according to the contents of independent claim 1

The vascular access device for haemodialysis of claim 1 can be permanently implanted in series to the pre-existing FAV, to the vein or artery or can hydraulically connect a vein to an artery. Herein it is disclosed a sterile package containing at least a single-use device of collecting and/or injecting blood in an arteriovenous fistula , that can be opened and used in each haemodialysis session hydraulically connecting the connecting element of the single-use device to an inlet tube of blood or to an outlet tube of blood of a haemodialysis apparatus, by acting on the flow regulating and/or intercepting device to enable the flow of the cannula and inserting the first end of the medical cannula (possibly piercing, with a needle, the patient's skin only at the second end of the second tubular element of the second conduit) through the second and first conduit of the vascular access device implanted according to the invention. As the only body element that is pierced is the skin, which easily heals, in particular if treated with appropriate fistula pressure pads comprising silver and/or another disinfecting agent and/or a healing agent, the pain caused to the patient during the haemodialysis sessions is significantly reduced. It is further clear that there is never a risk of passing from side to side of the vein, artery or arteriovenous fistula during the haemodialysis sessions, as the insertion of the collecting or return device from and into the vein is achieved by cannulation and takes place at the vascular access device and with the inserting of a free end of a medical cannula for cannulation. This end is by definition non-cutting and is sufficiently flexible to conform and insert, first in the second conduit and then in the first conduit of the vascular access device for haemodialysis. Consequently there is no risk of damage to the FAV. Additionally, the first conduit can have the same internal diameter as the FAV, the vein or the artery and the cannula can have, as a function of the relative thickness, a diameter of less by 0.1-0.2 mm to the internal diameter of the first conduit, i.e. an internal diameter of 2.0-4.0 mm, therefore much greater than the diameter of the fistula needle, which is 1.6 mm. This enables notably increasing the quantity of blood collected and returned into the FAV during haemodialysis. Therefore, by appropriately dimensioning the filtering surface of the semi-permeable membrane of the haemodialysis apparatus on the basis of the blood flow that can flow into the cannula having the internal diameter of 2.0 -4.0 mm, it will be possible to significantly limit the times and costs of personnel relative to a single haemodialysis treatment while increasing personnel productivity.

Note that the use of the vascular access healing device enables, by inserting it at the point of vascular access of the body and in the second conduit and/or in the third conduit, healing device the valve access about the cylinder of the healing device. In fact, by leaving the healing device for a few days in the site, removing it for periodic medication of the vascular access (at least 2-3 times a week), and replacing it, after each medication, it is possible to obtain, in a relatively short time, a permanent vascular access having a permanently open healed vascular access hole. The vascular access hole opens on the second conduit and/or on the third conduit of the vascular access device of the invention to which is preferably connected, respectively, the first or second valve, which hydraulically close the respective second or third conduit. In this way blood loss can be drastically limited. Note that the permanent vascular access having a healed access hole is passed through by a cannula for cannulation, in particular the cannula comprised in the sterile package. It is therefore not necessary to use fistula needles to carry out the dialysis. Consequently the dialysis can also be carried out by an operator who is not highly specialised in dialysis as the inlet of the cannula in the permanent vascular access is enormously simplified with respect to the prior art and further facilitates the connection of the haemodialysis apparatus to the patient. Further, as there is no use of fistula needles, the discomfort and pain felt by the patient is reduced, as is the probability of damaging veins, arteries or the FAV.

According to the characteristics of the person to be dialysed, the time required for obtaining the permanent vascular access with the permanently open healed vascular access hole can vary from 4 to 12 weeks, or from 8 to 10 weeks. In this period and in any case, in a case where the healing device is not used, until the implant of the vascular access device has matured, (i.e. is ready to be used) dialysis is not carried out or is carried out using a central venous catheter, which will then be eliminated. Additionally to the advantages set out in the foregoing, the vascular access device for haemodialysis of claim 1 further and advantageously enables choosing the blood vessel in which to implant it; in fact it can be implanted in series to an artery (A) to a vein, to a pre-existing arteriovenous fistula or, more preferably, it can be implanted between an artery and a vein to constitute in itself an artificial arteriovenous fistula. In this case, with a single operation, a permanent vascular access and outlet and an artificial arteriovenous fistula are created, thus limiting the number of operations to which the patient has to be subjected, as well as the consequent risks of post-operatory complications and the discomfort suffered by the patient.

### BRIEF DESCRIPTION OF THE DRAWINGS

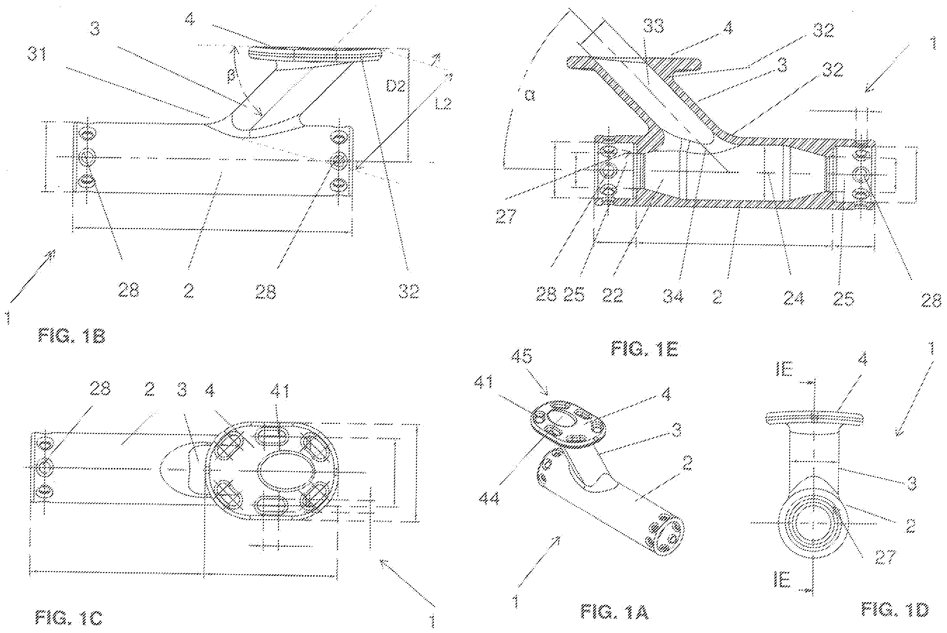

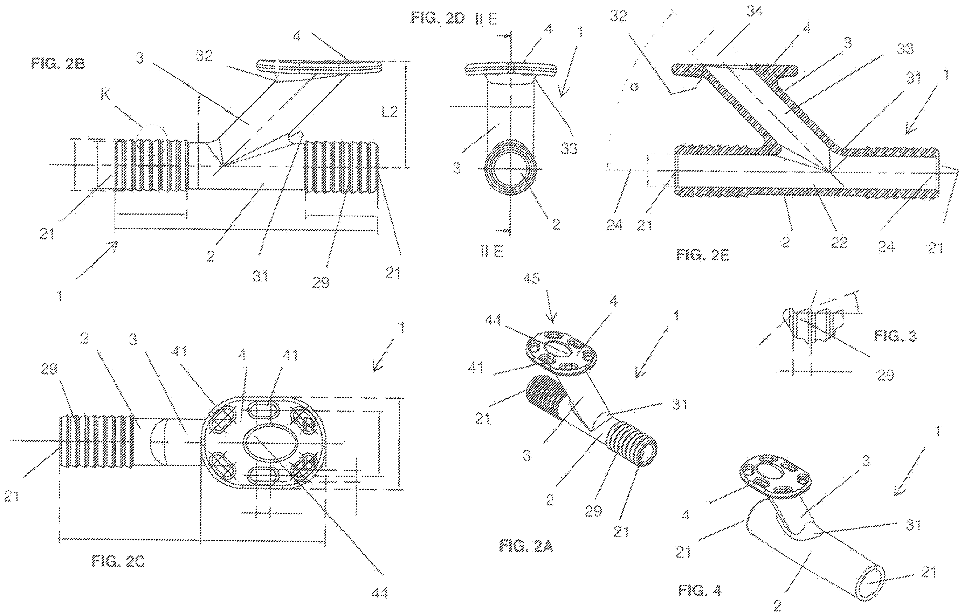

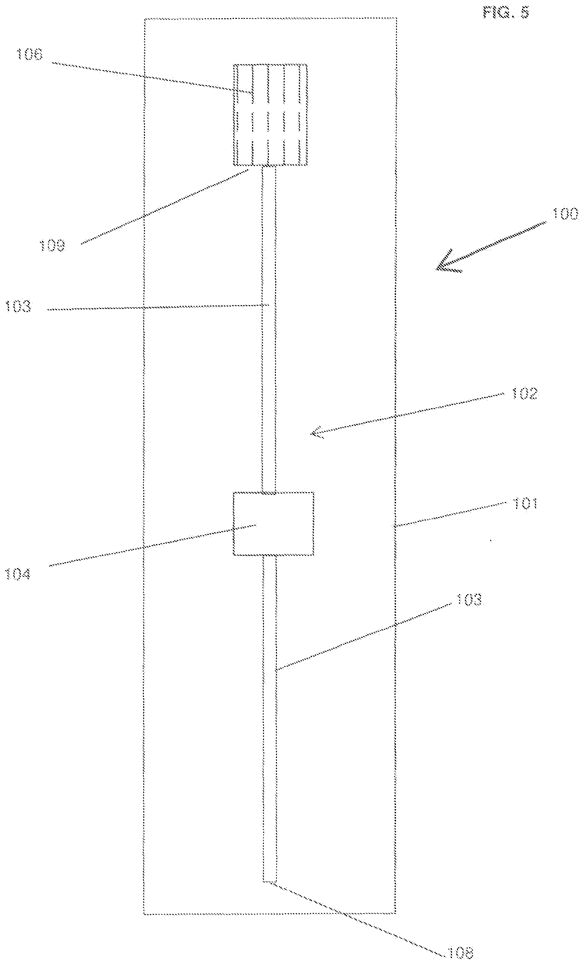

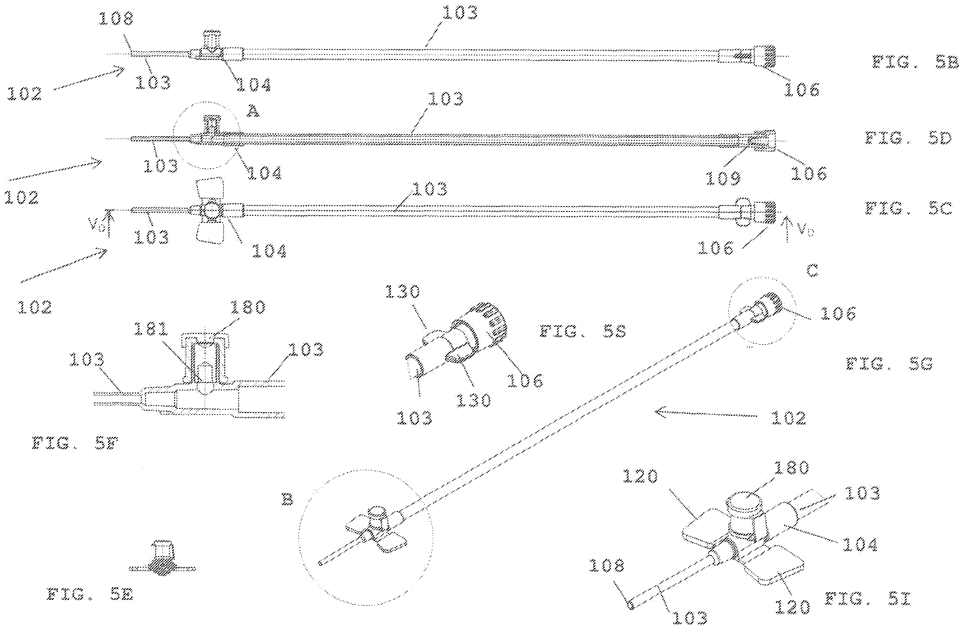

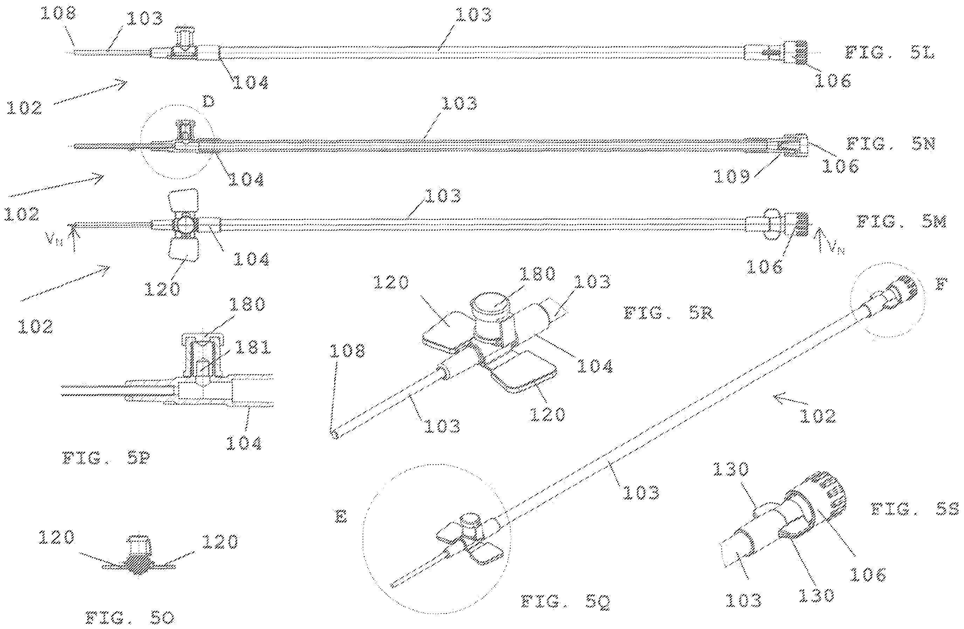

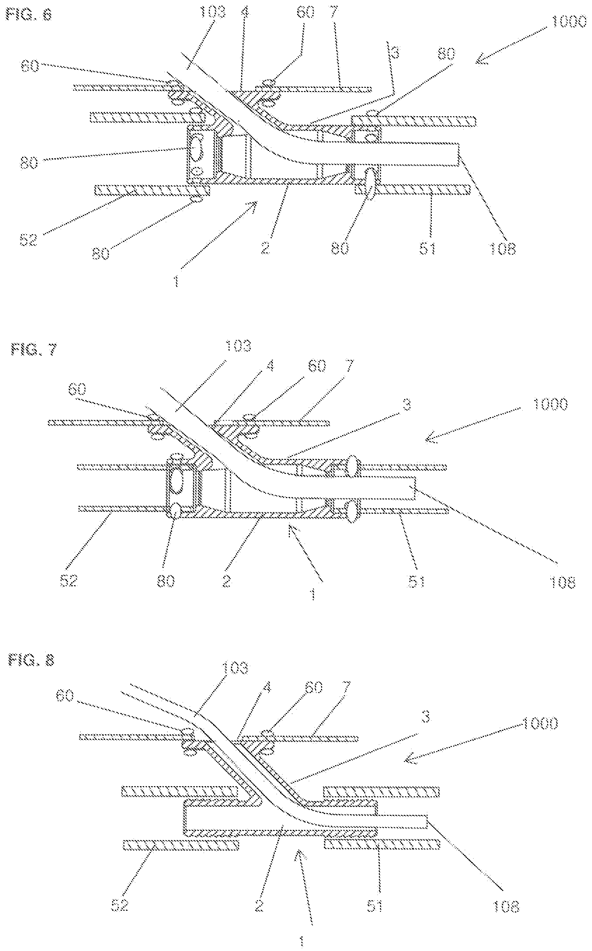

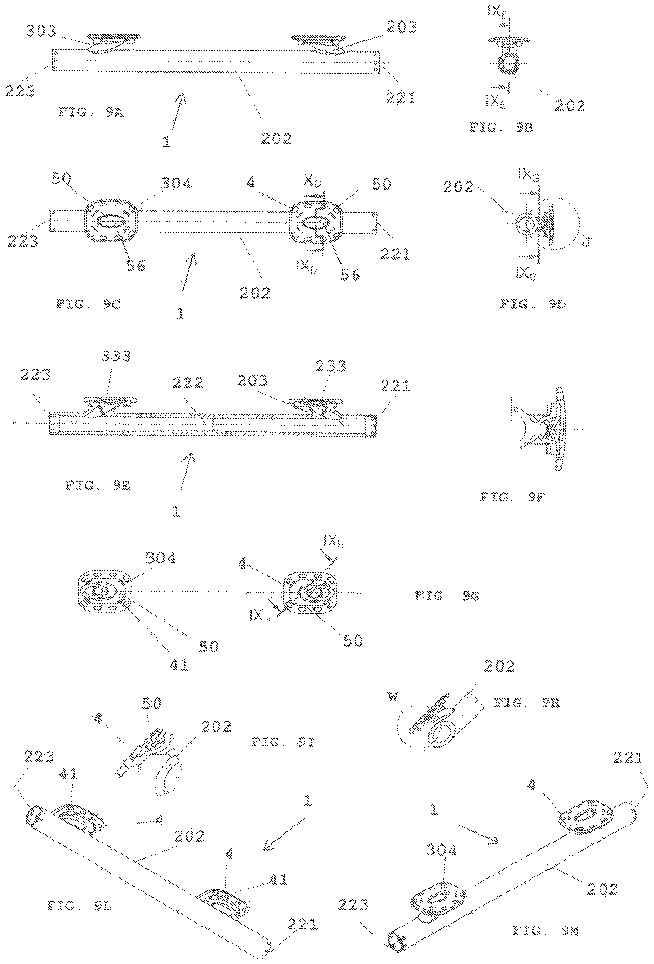

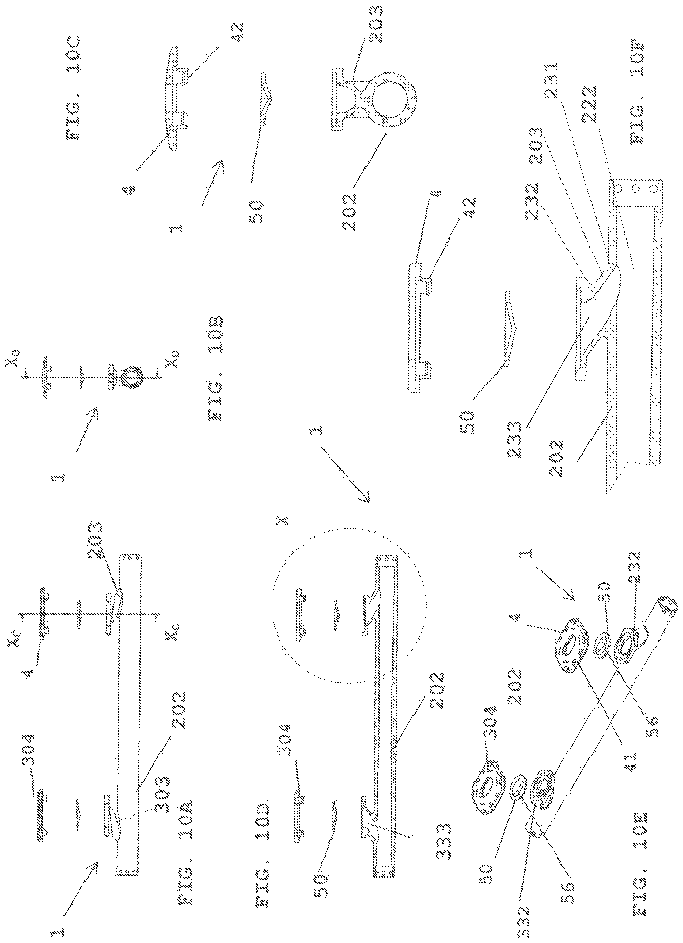

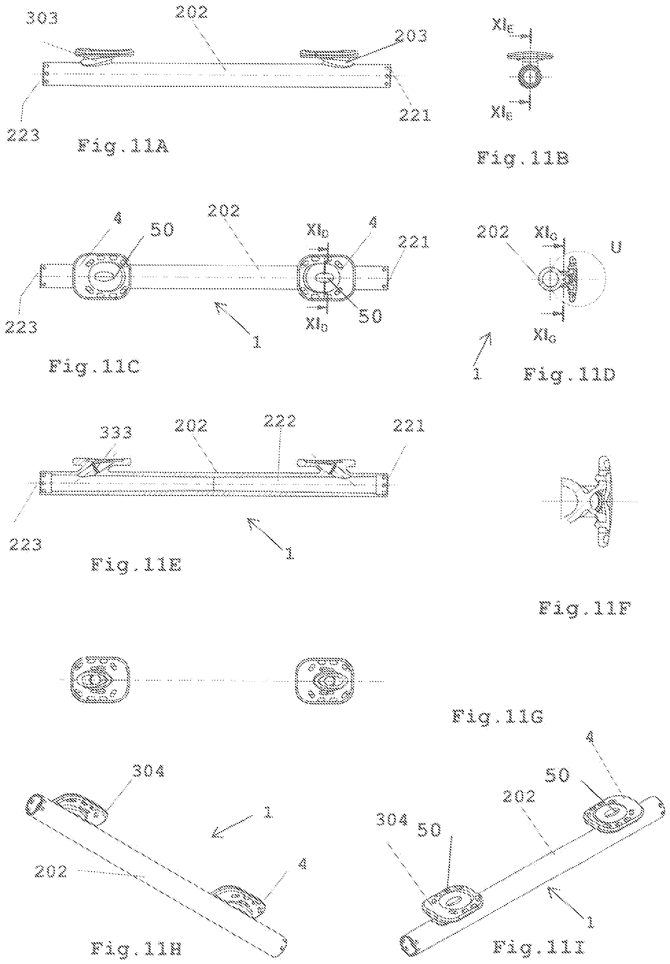

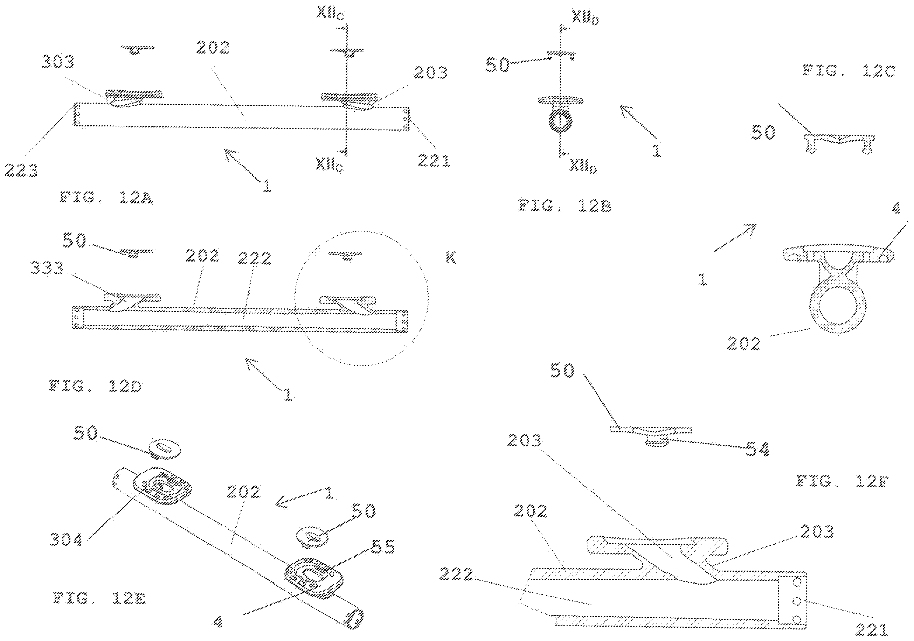

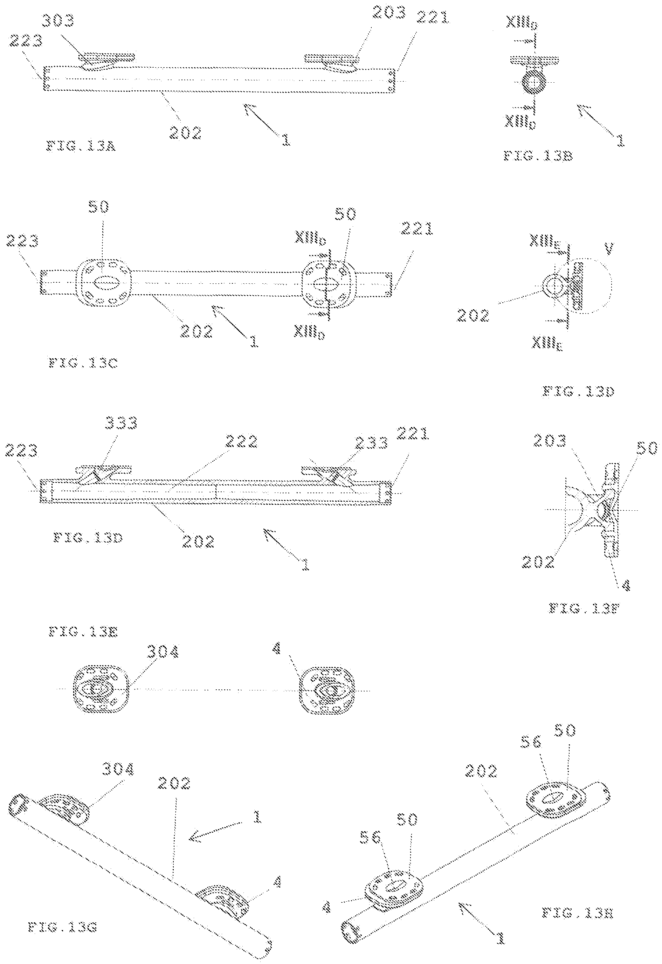

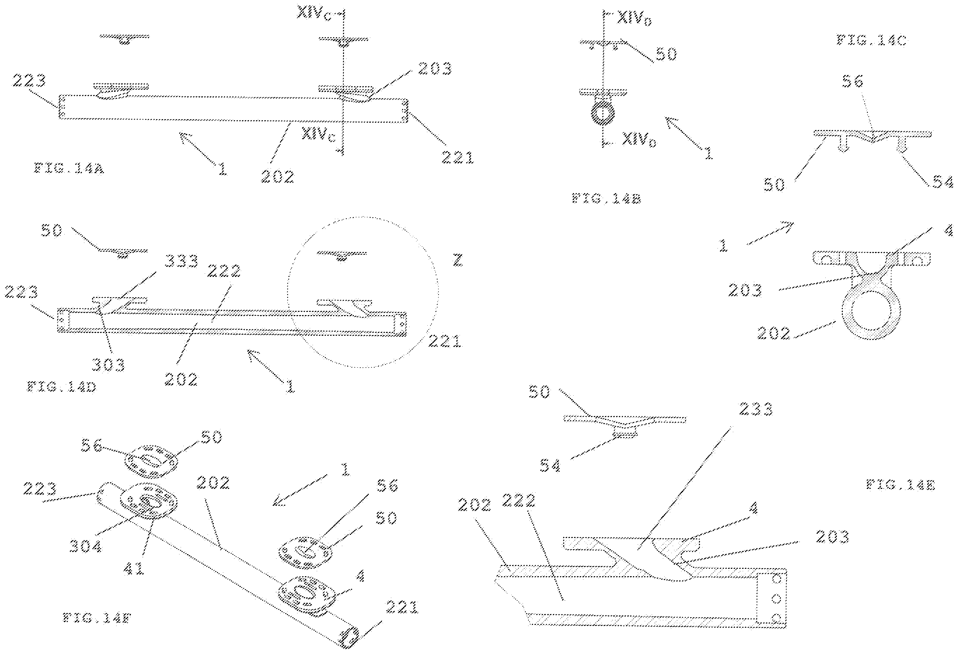

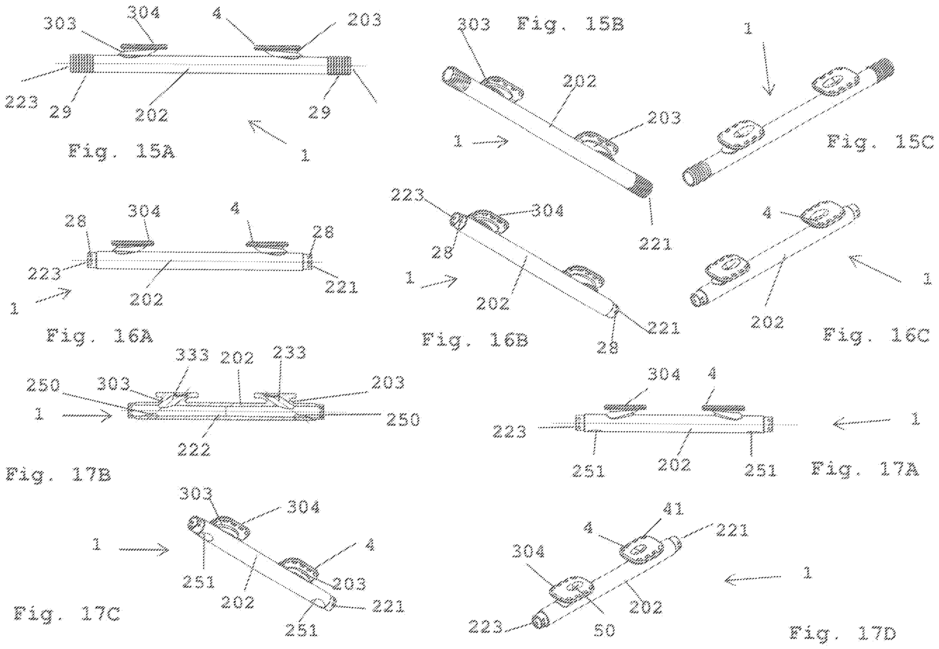

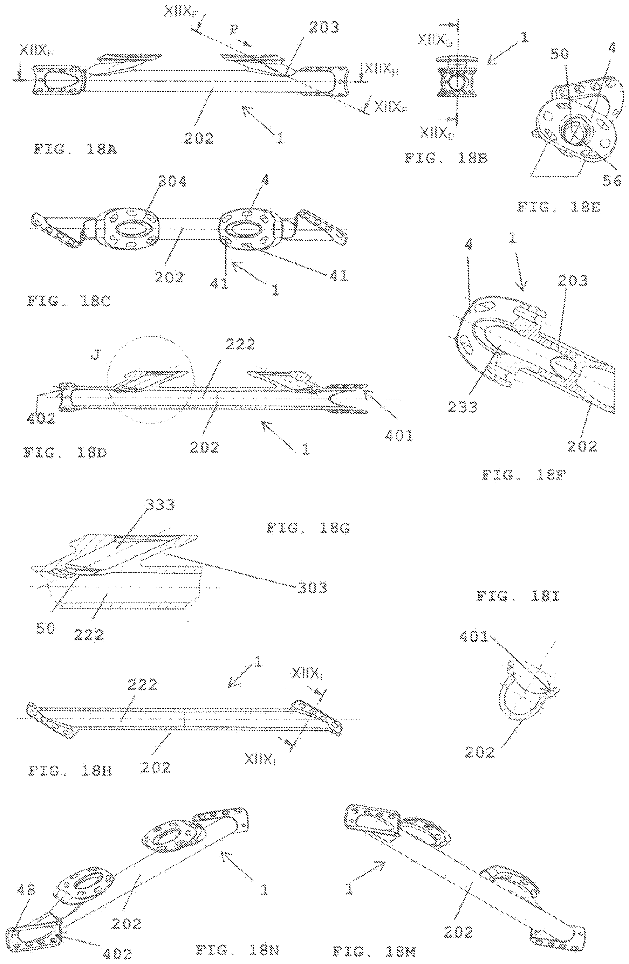

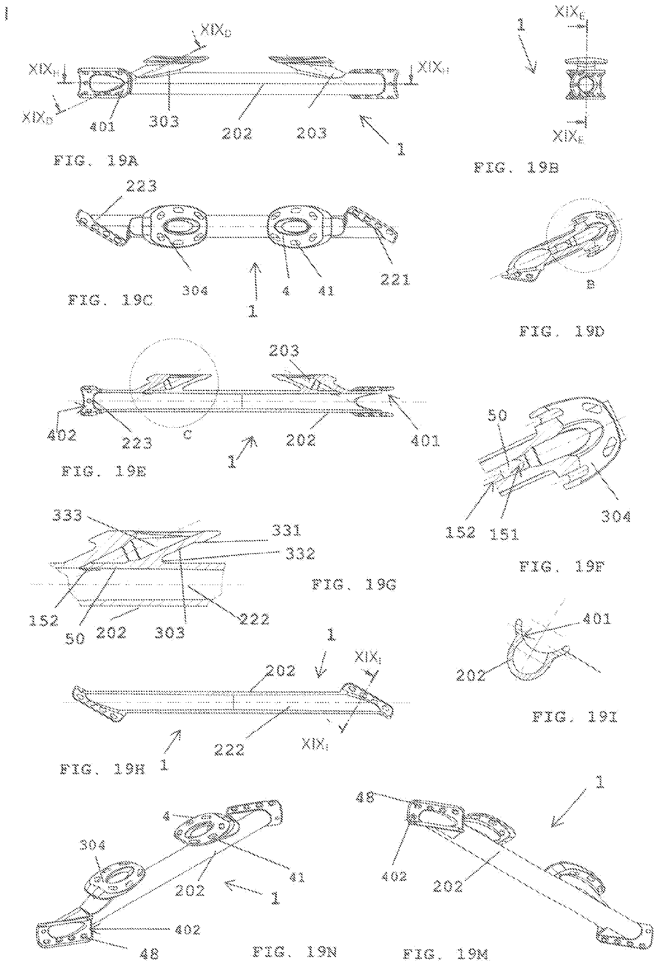

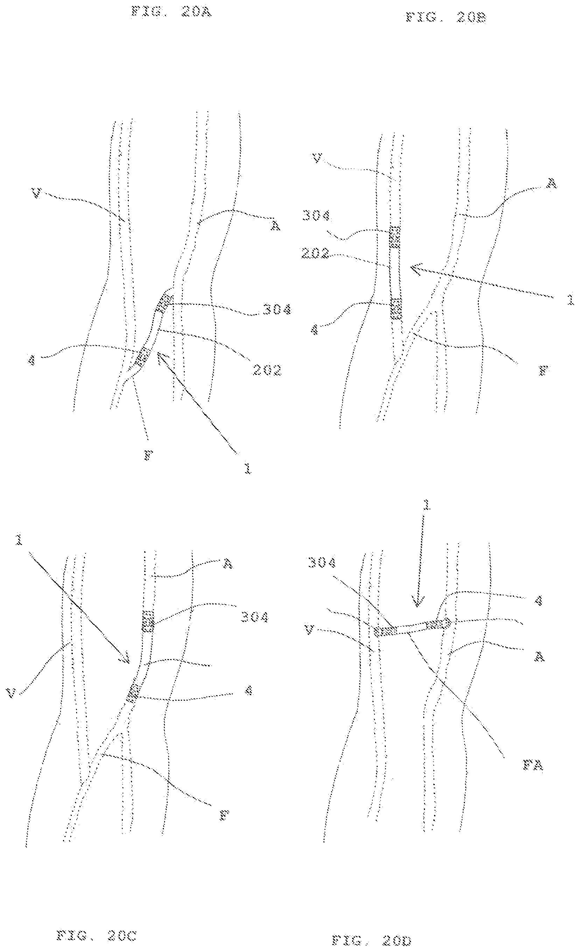

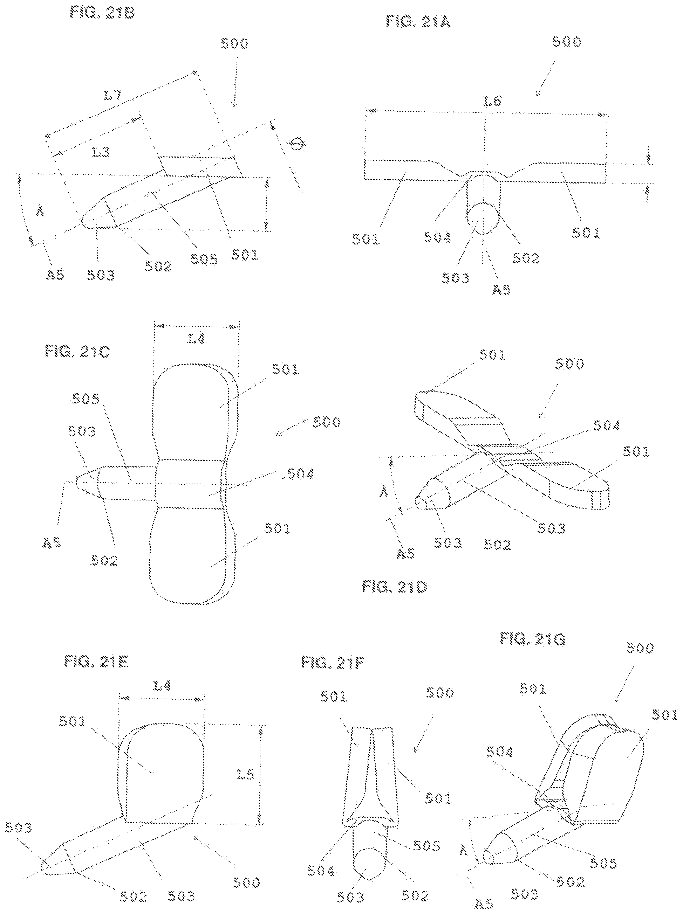

The characteristics of the invention will emerge in the following, which describes preferred but non-exclusive embodiments, with reference to the appended drawings, in which: figures 1A, 1B, 1C, 1D and 1E are schematic views, respectively perspective, lateral, from above, frontal, and in section with respect to plant IE-IE of figure 1D of a first embodiment of a vascular access device for haemodialysis according to the invention; figures 2A, 2B, 2C, 2D and 2E are schematic views, respectively perspective, lateral, from above, frontal, in section with respect to plant 2E-2E of figure 2D of a second embodiment of a vascular access device for haemodialysis according to the invention; figure 3 is a larger-scale view of detail K of figure 2B; figure 4 is a schematic perspective view of a third embodiment of a vascular access device for haemodialysis according to the invention; figure 5A is a view from above of a sterile package which is not part of the invention; figure 5B is a lateral view of a further embodiment of the single-use device of collecting and/or injecting blood which is not part of invention; figure 5C is a view from above of the single-use device of figure 5B; figure 5D is a section view according to axis V_{D}-V_{D} of figure 5C of the single-use device of figure 5B; figure 5E is a front view of the single-use device of figure 5B; figure 5F is a larger-scale view of detail A of figure 5D; figure 5G is a perspective view of the single-use device of figure 5B; figure 5I is a larger-scale view of detail B of figure 5G; figure 5H is a larger-scale view of detail C of figure 5G; figure 5L is a lateral view of a further embodiment single-use of the single-use device for collecting and/or injecting blood; figure 5M is a view from above of the single-use device of figure 5L; figure 5N is a section view according to axis V_{N}-V_{N} of figure 5M of the single-use device of figure 5L; figure 5O is a front view of the single-use device of figure 5L; figure 5P is a larger-scale view of detail D of figure 5N; figure 5Q is a perspective view of the single-use device of figure 5L; figure 5R is a larger-scale view of detail D of figure 5G; figure 5S is a larger-scale view of detail D of figure 5G; figure 6 is a schematic view in section of a first embodiment of a portion of a collection and/or injection system for haemodialysis according to the invention coupled to an arteriovenous fistula in a use configuration; figure 7 is a schematic view in section of a second embodiment of a portion of a collection and/or injection system for haemodialysis according to the invention coupled to an arteriovenous fistula in a use configuration; figure 8 is a schematic view in section of a third embodiment of a portion of a collection and/or injection system for haemodialysis according to the invention coupled to an arteriovenous fistula in a use configuration; figure 9A is a lateral view of a fourth embodiment of a vascular access device according to the invention; figure 9B is a rear view of the device of figure 9A, figure 9C is a view from above of the device of figure 9A, figure 9D is a section view of the device of figure 9A along plane IX_{D}- IX_{D} of figure 9C, figure 9E is a section view of the device of figure 9 along plane IX_{E}- IX_{E} of figure 9B, figure 9F is a larger-scale view of detail J of figure 9D; figure 9G is a section view of the device of figure 9A according to plane IX_{G}- IX_{G} of figure 9D, figure 9H is a section view of the device of figure 9A according to plane IX_{H}- IX_{H} of figure 9G, figure 9I is a larger-scale view of detail K of figure 9H, figures 9L and 9M are perspective views, respectively from below and above, of the device of figure 9A, figure 10A is an exploded view from above of the device of figure 9A, figure 10B is a rear view of the exploded device of figure 10A, figure 10C is a section view of the exploded device of figure 10A according to plane X_{C}- X_{C} of figure 10A, figure 10D is a section view of the exploded device of figure 10A according to plane X_{D}- X_{D} of figure 10B, figure 10E is a perspective view of the exploded device of figure 10A, figure 10F is a larger-scale view of detail X of figure 10D, figure 11A is a lateral view of a fifth embodiment of a vascular access device for haemodialysis according to the invention, figure 11B is a rear view of the device of figure 11A, figure 11C is a view from above of the device of figure 11A, figure 11D is a section view of the device of figure 11A according to plane XI_{D}- XI_{D} of figure 11C, figure 11E is a section view of the device of figure 11A according to plane XI_{E}- XI_{E} of figure 11B, figure 11F is a larger-scale view of detail K of figure 11D, figure 11G is a section view of the device of figure 11A according to plane XI_{G}-XI_{G} of figure 11D, figures 11L and 11M are perspective views, respectively from below and above, of the device of figure 11A, figure 12A is an exploded view of the device of figure 11A, figure 12B is a rear view of the exploded device of figure 12A, figure 12C is a section view of the exploded device of figure 12A according to plane XII_{C}- XII_{C} of figure 12A, figure 12D is a section view of the exploded device of figure 12A according to plane XII_{D}- XII_{D} of figure 12B, figure 12E is a perspective view of the exploded device of figure 12A, figure 12F is a larger-scale view of detail K of figure 12D, figure 13A is a lateral view of a sixth embodiment of a vascular access device according to the invention, figure 13B is a rear view of the device of figure 13A, figure 13C is a view from above of the device of figure 13A, figure 13D is a section view of the device of figure 13A according to plane XIII_{D}- XIII_{D} of figure 13C, figure 13E is a section view of the device of figure 13A along plane XIII_{E}- XIII_{E} of figure 13D, figure 13F is a larger-scale view of detail K of figure 13D, figures 13G and 13H are perspective views, respectively from below and above, of the device of figure 13A, figure 14A is an exploded view of the device of figure 13A, figure 14B is a rear view of the exploded device of figure 14A, figure 14C is a section view of the exploded device of figure 14A according to plane XIV_{C}- XIV_{C} of figure 14A, figure 14D is a section view of the device of figure 14A according to plane XIV_{D}- XIV_{D} of figure 14B, figure 14E is a larger-scale view of detail Z of figure 14D, figure 14F is a perspective view of the device of figure 14A, figures 15A, 15B, and 15C are, respectively, lateral and perspective views from below and above of a seventh embodiment of a vascular access device according to the invention figures 16A, 16B, and 16C are, respectively, lateral and perspective views from below and above of an eighth embodiment of a vascular access device according to the invention, figures 17A, 17B, 17C, and 17D are, respectively, lateral, longitudinal section and perspective views from below and above of a ninth embodiment of a vascular access device according to the invention; figure 18A is a lateral view of a tenth embodiment of a vascular access device according to the invention; figure 18B is a rear view of the device of figure 18A, figure 18C is a view from above of the device of figure 18A, figure 18D is a section view of the device of figure 18A according to plane XIIX_{D}-XIIX_{D} of figure 18B, figure 18E is a view of the device of figure 18A from side P, figure 18F is a section view of the device of figure 18A according to plane XIIX_{F}-XIIX_{F} of figure 18A, figure 18G is a larger-scale view of detail J of figure 18D, figure 18H is a section view of the device of figure 18A according to plane XIIX_{H}-XIIX_{H} of figure 18A, figure 18I is a section view of the device of figure 18A according to plane XIIX_{I}-XIIX_{I} of figure 18H, figures 18N and 18M are perspective views, respectively from below and above, of the device of figure 18A, figure 19A is a lateral view of an eleventh embodiment of a vascular access device according to the invention, figure 19B is a rear view of the device of figure 19A, figure 19C is a view from above of the device of figure 19A, figure 19D is a section view of the device of figure 19A according to plane XIX_{D}-XIX_{D} of figure 19B, figure 19F is a larger-scale view of detail B of figure 19D, figure 19E is a section view of the device of figure 19A according to plane XIX_{E}-XIX_{E} of figure 19B, figure 19G is a larger-scale view of detail C of figure 19E, figure 19H is a section view of the device of figure 19A according to plane XIX_{H}-XIX_{H} of figure 19A, figure 19I is a section view of the device of figure 19A according to plane XIIX_{I}-XIIX_{I} of figure 19H, figures 19N and 19M are perspective views, respectively from below and above, of the device of figure 19A;figures 20A, 20B, and 20C are views illustrating the positions in which a vascular access device according to the invention can be implanted, figures 21A, 21B, 21C, and 21D are schematic views, respectively, frontal, lateral, from above and perspective, of a healing device for valve access which is not part of the invention in first relative configuration thereof and figures 21E, 21F and 21G are schematic views, respectively, lateral, frontal and perspective, of the device of figure 2A in a second relative configuration.

### DESCRIPTION OF PREFERRED EMBODIMENTS

With reference to the figures, reference numeral (1) relates to a vascular access device for haemodialysis, permanently implantable in a patient having an arteriovenous fistula, (1) denotes a vascular access device for haemodialysis according to the invention, (with both single and double vascular access) permanently implantable in a patient, (102) relates to a single-use device of collecting and/or injecting blood, (500) denotes a healing device for valve access, (100) a sterile package and (1000) denotes a collection and/or injection system for haemodialysis according to the invention. As can be noted from the figures, the embodiments, from the first to the third, relate to a vascular access device according to the single access invention (see figures 1A-4 and 6-8), while the embodiments from the fourth to the eleventh relate to a vascular access device according to the invention which is double access as they enable a double vascular access (see figures 9-19N). The vascular access device of the double access invention can in fact be used both for the collection, from the patient, of the blood to be subjected to haemodialysis, and for the re-injection into the patient of the blood subjected to dialysis for example by collecting the blood from the second tubular element (203) and re-injecting the blood in the third tubular element (303), or vice versa. The single access vascular access devices, however, enable only one access, either in inlet or outlet, and therefore two of them have to be implanted for each patient.

According to the invention, the single access vascular access device (1) is a vascular access device (1) made of a biocompatible and sterilisable material, and comprises:
- a relative first tubular element (2) having a first and a second relative end (21) which are longitudinal, the first tubular element (2) defining a first conduit (22) which extends from the first to the second end (21);
- a relative second tubular element (3) having a first and a second relative end (31, 32) which are longitudinal, and defining a second conduit (33) which extends from the relative first to the second end (31, 32); wherein the first end (31) of the second tubular element (3) is fixed to the first tubular element (2) in an intermediate position between the first and the second end (21) of the second tubular element (3), (see figures 1A, 1B, 1E, 2A, 2B, 2E, and 4), so that the second conduit (33) is in fluid communication with the first conduit (22) and so that the second end (32) of the second tubular element (3) is on a first side with respect to the first tubular element (2);
- first fixing means (4) comprising a first plurality of through-holes (41) and being fixed to the first tubular element (202) and/or second tubular element (3) (preferably they are fixed to the second tubular element (3)) the first plurality of through-holes (41) being arranged on the first side, with respect to the first tubular element (2), and at the second end (32) of the second tubular element (3), wherein said vascular access device (1) is conformed and dimensioned to be implantable in an arm of a patient having an arteriovenous fistula for haemodialysis with the first conduit (22) interposed in series to the arteriovenous fistula, with respect to the blood flow therein, between a first portion (51) and a second portion (52) of the arteriovenous fistula, the first end (21) and the second end (21) of the first tubular element (2) being fixed, respectively to the first portion (51) and to the second portion (52) of the arteriovenous fistula, at a relative end of the relative portion (51, 52) and the second end (32) of the second tubular element (3) and with the first plurality of through-holes (41) arranged subcutaneously at an implant skin region (7) in order to enable fixing of the first plurality of through-holes (41) to the implant skin region (7) by means of a first plurality of suture stitches (60), and a consequent fixing of the second end (32) of the second conduit (33) to the implant skin region (7). Note that, for the second conduit (33) to be in fluid communication with the first conduit (22) and so that the second end (32) of the second tubular element (3) is on a first side with respect to the first tubular element (2), the first end (31) of the second tubular element (3) cannot be fixed to the first tubular element (2) in an intermediate position between the first and the second end (21) of the second tubular element (3). In fact, the first end (31) of the second tubular element (3) is fixed in an intermediate position between the first and the second end (21) of the first tubular element (2) as can be observed in figures 1A, 1B, 1E, 2A, 2B, 2E and 4.

The vascular access device (1) of the invention, of the double access type, like those illustrated in figures 9-19, is a vascular access device (1) for haemodialysis permanently implantable in a patient, and is made of a biocompatible and sterilisable material, and comprises:
- a relative first tubular element (202) having a first and a second relative end (221, 223) which are longitudinal, the first tubular element (2) defining a first conduit (222) which extends from the first to the second end (221, 223);
- a relative second tubular element (203) having a first and a second relative end (231, 232) (See figure 10F), which are longitudinal, and define a second conduit (233) which extends from the relative first to the second end (231, 232); wherein the first end (231) of the second tubular element (203) is fixed to the first tubular element (202) in proximity of the first end (221) of the first tubular element (202), so that the second conduit (233) is in fluid communication with the first conduit (222) and so that the second end (232) of the second tubular element (203) is on a first side with respect to the first tubular element (202);
- a relative third tubular element (303) having a first and a second relative end (331, 332), which are longitudinal, the relative third tubular element (303) defining a third conduit (333) which extends from the relative first to the second end (331, 332) (See figure 19G); wherein the first end (331) of the third tubular element (303) is fixed to the first tubular element (202) in proximity of the second end (223) of the first tubular element (202), so that the third conduit (333) is in fluid communication with the first conduit (222) and with the second conduit (233) and so that the second end (332) of the third tubular element (303) is on the first side with respect to the first tubular element (202); and
- first fixing means (4) comprising a first plurality of through-holes (41) and being fixed to the first tubular element (202) and/or second tubular element (203) the first plurality of through-holes (41) being arranged on the first side, with respect to the first tubular element (202), and at the second end (232) of the second tubular element (203),
- second fixing means (304) comprising a second plurality of through-holes (41) and being fixed to the first tubular element (202) and/or third tubular element (303) the second plurality of through-holes (41) being arranged on the first side, with respect to the first tubular element (202), and at the second end (332) of the third tubular element (303), wherein the vascular access device (1) is conformed and dimensioned to be implanted in a patient, preferably a patient's arm, in:
- a first position which is at an artery (A), at a vein (V), or at a pre-existing arteriovenous fistula (F) (see respectively figures 20C, 20B and 20A) the first conduit being interposed in series with respect to the blood flow, respectively in the artery (A), in the vein (V) or in the pre-existing arteriovenous fistula (F) between a first portion and a second portion, respectively, of the artery (A), vein (V), or pre-existing arteriovenous fistula (F), with the first end and the second end (221, 223) of the first tubular element (202) fixed respectively to the first portion (51) and second portion (52), and/or
- in a second position, between the artery (A) and the vein (V) (see figure 20D), with the relative first or second end (231, 232) of the first tubular element (202) fixed to the artery (A) with the remaining of the first or second relative end (231, 232) of the first tubular element (202), which is not fixed to the artery (A), fixed to the vein (V) in such a way that the first conduit (222) hydraulically connects the artery (A) to the vein (V) to constitute an artificial arteriovenous fistula (FA), wherein, in the first and second position, the second end (232) of the second tubular element (203) and the second end (332) of the third tubular element (303), the first and second plurality of through-holes (41) arranged subcutaneously in an implant skin region (7) in order to enable fixing of the first and second plurality of through-holes (41) to the implant skin region (7) by means of a corresponding first and a corresponding second plurality of suture stitches (60), and the consequent fixing, respectively, of the second end (232) of the second conduit (233) and the second end (332) of the third tubular element (303) to the implant skin region (7).

The double access vascular access device is not only implantable in series to a blood vessel (vein, artery A and pre-existing arteriovenous fistula), but also advantageously enables carrying out the following surgical method for implanting the device which comprises following steps:
predisposing the double access vascular access device of the invention; carrying out a surgical incision on a wall of a vein (V) at an implantation site; carrying out a surgical incision on a wall of an artery (A) at an implantation site; hydraulically connecting the vein and the artery via a first conduit 222, which is hydraulically connected at the relative ends, respectively to a second conduit (233) and to a third conduit (333); fixing the relative first or second end (231, 232) of the first tubular element (202) fixed to the artery (A) at the relative surgical incision; and fixing, with the remaining of the first or second relative end (231, 232) of the first tubular element (202), which is not fixed to the artery (A) at the relative surgical incision, to the vein (V) in such a way that the first conduit (222) hydraulically connects the artery (A) to the vein (V) to constitute an artificial arteriovenous fistula (FA), arranging the second end (232) of the second tubular element (203) and the second end (332) of the third tubular element (303), the first and second plurality of through-holes (41) arranged subcutaneously in an implant skin region (7); and fixing the first and second plurality of through-holes (41) to the implant skin region (7) by means of a corresponding first and a corresponding second plurality of suture stitches (60).

With the aim of obtaining a permanent vascular access having a permanently open healed vascular access hole the applicant has also developed a healing device (500) of a vascular access, comprising:
- a relative insertion portion (502) which is insertable internally of a vascular access and which comprises: a cylinder (505) having a relative longitudinal development axis: a first and a second relative end which are longitudinal; and a distally tapered tip (503) (preferably coaxial to the cylinder) fixed to the first end of the cylinder (505); wherein the insertion portion is insertable in the second conduit (33, 233) or in the third conduit (333) of a vascular access device (1) for haemodialysis according to any one of claims 4, 7, 8, 9 and 10 from the relative second end (32, 232, 332) so as to occlude exclusively a portion of the second conduit (33, 233) or the third conduit (333) comprised between, respectively, the first valve (50) and the first plate (4) or between the second valve (50) and the second plate (304);
- a relative connection portion (504) fixed to the second end of the cylinder (505); and
- a relative first and a relative second gripping tab (501), fixed to the connection portion (504) on opposite sides thereof and on opposite sides with respect to the cylinder axis and with possibility of relative movement with respect to the connection portion (504), in order to be able to assume: a first relative configuration in which the first and the second gripping tab (501) are substantially coplanar (see figures 21A-21D); and a second configuration in which the tabs are opposite one another (see figures 21E-21F), wherein in both the first and the second configuration the first and the second gripping tab (501) are not insertable in the second conduit (33, 233) or third conduit (333).

In a relative embodiment of the vascular access healing device herein disclosed, the relative cylinder is inclined with respect to the connection portion by an angle (λ) (see respectively figures 21B, 21D and 21G) identical to angle β (See figure 1A) with which the second tubular element (3, 203) or the third tubular element (303) is inclined with respect, respectively, to the first plate (4) or the second plate (304).

With reference to figures 21A-21G, the length L3 of the insertable portion (502) is preferably comprised between 2 or 2.5 mm and 5 mm to avoid contacting the valve (50) it is preferably 1-3 mm. The gripping tabs (501) can have following dimensions: L4 comprised between 3 and 6 mm, preferably 4.5 mm and L5 comprised between 4.5 and 6 mm, preferably 5 mm. The diameter Θ of the cylinder obviously depends on the dimensions of the second and third conduit, but can advantageously be 1.5 - 2 mm, preferably 1.75 mm. The width L6 of the healing device (500) of the vascular access can be 10-15 mm, preferably about 12.5 mm, while the length L7 can be of 7-10 mm, preferably about 8.5 mm.

The sterile package (100) which is not part of the invention contains at least a single-use device (102) of collecting and/or injecting blood in an arteriovenous fistula constituted by:
- a medical cannula (103) for cannulation having a relative first end (108) and a relative second end (109) wherein the first end (108) is free and cannulable in a pre-existing arteriovenous fistula (F), an artery (A), or a vein (V) of a patient through the second conduit (33, 233) and the first conduit (22, 222) or through the third conduit (333) and the first conduit (222) of both a double and single access device;
- a flow regulating and/or intercepting device (104) predisposed to regulate and/or interrupt a flow of a liquid in the medical cannula (103); and
- a connecting element (106), fixed to the second end (109) of the medical cannula (108) to enable hydraulic connection to an inlet tube of blood or to an outlet tube of blood of a haemodialysis apparatus;
- optionally an access element arranged between the first end (108) of the cannula and the regulating and/or intercepting device (104) and defining an access channel (181) (see figures 5F and 5P) hydraulically connected to the first end (108) of the cannula; and reversible closing means (180) for reversibly closing the access channel (181) at a distal end of the access element in order to enable injection of a liquid into the access channel (181) and to cause the liquid to flow towards the first end (108) of the medical cannula (103);
- optionally a first gripping element (120), arranged between the first end (108) and/or the regulating and/or intercepting device (104), and/or a second gripping element (130), arranged proximally to the connecting element (106). Obviously the package also comprises a relative wrapping (101), sealed and made of a suitable material which enables sterilisation of the contents thereof, for example the wrapping (101) can be made with a sheet of a material that is permeable to ultra-violet rays and/or to ionising radiations (gamma and/or beta rays) and/or to microwaves. By appropriately selecting both the relative conformations and dimensions of the vascular access device (1) and the single-use device (102) of collecting and/or injecting, contained in the sterile package (100), it is sufficient to open the package at each haemodialysis session so as to easily obtain a collection and/or injection system (1000) for haemodialysis which comprises both a vascular access device (1) and the single-use device (102) of collecting and/or injecting, where the first free end (108) of the medical cannula (103) is free and cannulable in an arteriovenous fistula, a vein or an artery of a patient, through the second conduit (33, 233) and the first conduit (22, 222) and/or through the third conduit (333) and the first conduit (22, 222) of the vascular access device (1). The system 1000 enables attaining the above-mentioned aims, with respect to the prior art.

A collection and/or injection system (1000) for haemodialysis, according to the invention, thus comprises a vascular access device (1) for haemodialysis according to the invention which can be double or single access and at least a single-use device (102) of collecting and/or injecting blood in an arteriovenous fistula (F), a vein (V) or an artery (A) constituted by:
- a medical cannula (103) for cannulation having a relative first end (108) and a relative second end (109) wherein the first end (108) is free and cannulable in a pre-existing arteriovenous fistula (F), an artery (A), or a vein (V) of a patient through the second conduit (33, 203) and the first conduit (22, 222) or through the third conduit (333) and the first conduit (22, 222) of a vascular access device (1);
- a flow regulating and/or intercepting device (104) predisposed to regulate and/or interrupt a flow of a liquid in the medical cannula (103); and

- a connecting element (106), fixed to the second end (109) of the medical cannula (108) to enable hydraulic connection to an inlet tube of blood or to an outlet tube of blood of a haemodialysis apparatus;
- optionally an access element arranged between the first end (108) of the cannula and the regulating and/or intercepting device (104) and defining an access channel (181) (see figures 5F and 5P) hydraulically connected to the first end (108) of the cannula; and reversible closing means (180) for reversibly closing the access channel (181) at a distal end of the access element in order to enable injection of a liquid into the access channel (181) and to cause the liquid to flow towards the first end (108) of the medical cannula (103); and
- optionally a first gripping element (120), arranged between the first end (108) and/or the regulating and/or intercepting device (104), and/or a second gripping element (130) arranged proximally to the connecting element (106); and
- wherein the collection and/or injection system (1000) can comprise optionally at least one healing device (500) of a vascular access, preferably a plurality of the healing devices (500), and advantageously from eight to thirty.

In the case of the double access vascular access device it is preferable for the system to comprise two single access devices (102) for collecting and/or injecting.

Note that the access element and the closing means enable aspiration of any blood clots present in the access device, both single and double access. Additionally, they enable injecting an anticoagulant into the first conduit (22, 222). This anticoagulant can be heparin, for example heparin locks, sodium citrate, urokinase and mixtures thereof.

To improve the sterilisation and mechanical resistance of the vascular access device (1), it is preferably made of a metal, more preferably it is titanium, although the invention can include being constructed also using biocompatible and sterilisable plastic materials and not resorbable by the body, as described in the following. With the aim of improving the fixing of the second end (32, 232) of the second tubular element (3, 203) and the second end (332) with respect to the third tubular element (303), when present in the vascular access device for haemodialysis, to the skin region (7) for implant, the first and second plurality of through-holes (41) can be constituted by two through-holes (41), preferably arranged on the opposite side, respectively, to the second conduit (33, 232) and the conduit (333). In a case where it is constituted by three through-holes it is preferable to arrange them about the second conduit (33, 233) and the third conduit (333) at an angular distance of 120°, if a greater number of through-holes (41) is included, they can be arranged along a closed loop which surrounds the second conduit (33, 233) at the second end (32, 232) of the second tubular element (3, 203) (See figures 1C and 2C). They can also preferably also be angularly equidistant. The through-holes (41) are preferably at least six in number. The same also applies to the holes of the second end (332) of the third tubular element (303), when the latter is present. To advantageously facilitate the insertion of the medical cannula (103) into the second (33) and the first conduit (22, 222), and/or into the first conduit (222)and into the third conduit (333) at least one of the two, between the cannula and the conduit, and preferably both, have relative oval internal sections, or, more preferably, circular. The conduits (22, 33) advantageously have cylindrical inner walls.

Obviously the first (2, 202) and second tubular element (3, 203) have relative longitudinal development axes (24, 34) which are incident to one another and define two angles of incidence, one supplementary to the other. This also applies to the first and third tubular element, in the case of the double access vascular access device. The preferred embodiments of the vascular access device (1) for haemodialysis are those in which the first (2) and second tubular element (3) have a relative longitudinal development axis incident to one another with a minimum angle (α) that is acute. This angle can preferably be comprised between 25° and 60°, more preferably between 25° and 50° and still more preferably between 28°- 30° or 43°- 48°, 25° and 45° and/or (preferably and) wherein, when the relative third tubular element (303) is present, the first tubular element (202) and third tubular element (303) have a relative longitudinal development axis incident to one another with a minimum angle (α) comprised between 25° and 50°, preferably 25°. The minimum angle (α) can advantageously be 25°, 35° or 45°. Obviously, as a function of the angle (α) the length (L2) of the second tubular element (3) measured will have to be appropriately dimensioned, obviously along the relative longitudinal development axis, in order to dimension the vascular access device (1) so that by fixing the first tubular element (2) to the FAV, the second end (32) of the second tubular element (3) is under the skin (7). However, the surgeon, on the basis of the physical characteristics of the patient (gender, body type, muscle mass and fatty mass), will be perfectly able to choose a vascular access device (1) that is suitable for implanting in a patient, with the relative first tubular element (2) in series to the FAV and the second end (32) of the second tubular element (3) under the skin (7).

In an embodiment of the vascular access device (1) for haemodialysis of the invention, the first fixing means (4) can originate from the second end (32, 232) of the second tubular element (3, 203) and/or (preferably and), when present, the second fixing means (304) can originate from the second end (332) of the third tubular element (303), for example, the first and/or second fixing means (4) can be constituted by a plurality of arm braces, not illustrated, arranged spoke-fashion with respect to the second conduit (33), where each arm-band comprises one or more of the through-holes of the first plurality of through-holes (41).

It is particularly preferable for the first fixing means (4) to comprise a plate (4) having a relative main through-hole (44), wherein the first plurality of through-holes (41) is arranged peripherally with respect to the main through-hole (44), wherein the plate (4) is fixed at a relative first face to the second end (32) of the second tubular element (3, 203) so that the second conduit (33, 233) is accessible from the relative main through-hole and/or (preferably and), when present, for the second fixing means to comprise a second plate (304) having a relative main through-hole, wherein the second plurality of through-holes (41) is arranged peripherally with respect to the relative main through-hole and wherein the second further plate (304) is fixed at a relative first face to the second end (332) of the third tubular element (303) so that the third conduit (333) is accessible from the relative main through-hole (44). This simplifies the realisation of the vascular access device (1). The main through-hole (44) advantageously has the same section as the second conduit (33) at the second end and they coincide, which facilitates the insertion of the medical cannula (103) into the first conduit (22).

In an advantageous aspect of the invention, the plate (4) is fixed to the second tubular element (3) with a relative second face to the second end (45) opposite the relative first face and arranged along a plane that is parallel to the longitudinal development axis (24) of the first tubular element (2, 202) and/or (preferably and), when present, the second plate (304) is fixed to the third tubular element (303) by a relative second face (45) opposite the relative first face and arranged along a plane that is parallel to the longitudinal development axis (24, 224) of the first tubular element (2, 202). As illustrated in the embodiments of figures 9 -19 the second and third tubular element (203, 303), are preferably arranged in such a way that the relative longitudinal development axes cross from the first side of the first tubular element (202) as this facilitates blood collections to be subjected to dialysis and the return of the dialysed blood into the patient. The second face (44) is destined to internally face the skin (7) and is preferably arranged at a relative distance (D2), with respect to the longitudinal development axis (24) of the first tubular element (2, 202) comprised between 5 and 10 mm, preferably between 6.5 and 8.0 mm. This distance (D2) also obviously depends on the minimum angle (α) and the above considerations are valid relative to the dimensioning and the surgeon's choice regarding the vascular access device (1).

With the aim of easily fixing the vascular access device (1) for haemodialysis, both with single and double access, when the vascular access is to be permanently implanted in series to the pre-existing FAV, to the artery (A) or to the vein (V), the first end and the second end (21, 221, 223) of the first tubular element (2, 202) can advantageously each comprise a third plurality of though-holes (28) (see figures 1A-1C, 6 and 16A-17D), arranged along a relative transversal fixing sector to enable fixing the first and the second end (21, 221, 223) of the first tubular element (2, 202) on an end, respectively, of the first portion (51) and the second portion (52) of the arteriovenous fistula by means of a third plurality of suture stitches (80) visible in figure 6. The suture can be carried out with the end of the first and second portion of artery (A), vein (V) or pre-existing arteriovenous fistula (F) arranged externally (as in figure 6) or internally of the first and/or second end of the first tubular portion. In this latter case, it is preferable that at least one from between the first end and the second end (21, 221, 223) of the first tubular element (2, 202), preferably both (as illustrated in figure 1E) each comprise a relative internal housing defined by the first conduit (22, 222) and comprising a relative internal annular abutment surface (27) for abutting (preferably entirely), when fitted on the end, respectively, of the first portion (51) and second portion (52) of the artery (A), the vein (V) or the pre-existing arteriovenous fistula (F), a terminal annular surface of the end, respectively, of the first portion (51) and the second portion (52) of the arteriovenous fistula (see figure 7 and 20A) of the artery A (See figure 20C) or vein (See figure 20B). This not only improves the fixing but also prevents, once the free end (108) of the medical cannula (103) has been inserted in the second conduit (33) and in the first conduit (22), during the cannulation in a portion of the artery (A), the vein (V) or the pre-existing arteriovenous fistula (F) of the free end (108), the cannula from striking against the terminal annular surface of the end portion. Obviously if the housing (25) is present only in one from between the first end and the second end (21, 221, 223) of the first tubular element (2, 202), this will be the end opposite the minimum angle (α).

Preferably, the third plurality of through-holes (28) comprises at least 4, preferably at least 6 and more preferably at least 8. They are advantageously arranged angularly equidistant. The through-holes (41, 28) of the first and second and third plurality of though-holes must obviously have a dimension and a conformation such as to enable crossing by a surgical suture needle. During the suturing operation of the first plurality and second plurality of through-holes (28), respectively, to the skin (7) and to the two portions of the pre-existing FAV (F), of the vein (V) or the artery (A) cut by the surgeon, the surgeon will make each of the suture stitches pass through a different through-hole.

In a further fixing mode of the first and second end (21) of the first tubular element (2, 202) to the two portions of the pre-existing FAV (F), vein (V) or cut artery (A), the ends can be fixed to an end, respectively, of the first portion (51) and the second portion (52) of the pre-existing arteriovenous fistula of the vein (V) or the artery (A) using surgical glue (not illustrated).

For the purpose of improving the fixing with surgical glue of the first and/or the second end of the first tubular element (2, 202) each can comprise a relative transversal fixing sector having a relative irregular external surface, for example comprising a plurality of concavities, slots or grooves. In a preferred aspect of the invention the relative transversal fixing sector has a relative plurality of external annular grooves (29) (see figures 2A-2E and 3) coaxial to the longitudinal development axis (24) of the first tubular element (2, 202) to improve the fixing by surgical glue of the first and second end (21) of the first tubular element (2, 202) to an end, respectively, of the first portion and the second portion of the pre-existing arteriovenous fistula of the vein (F) or the artery (A).

Considering the average body size of the patients, it is preferable for the vascular access device (1) for haemodialysis of the invention, single or double access, to have the following characteristics, taken alone or in combination:
- the first conduit (22, 222) has a relative minimum internal radius comprised between 2 and 5 mm, preferably 2.5 - 4.0 mm, and/or a relative external radius comprised between 3 and 7 mm, 4mm and 6.0 mm, preferably between 4 and 6.0 mm and/or
- the second conduit (33, 233) and/or the third conduit (333), when present, has a relative minimum internal radius comprised between 2.0 and 5.0 mm, preferably 2.5 - 4 mm, and/or a relative external radius comprised between 3.0 and 6.0 mm, preferably between 3.5 and 5.0 mm and/or
- the first plurality of through-holes (41) and/or the second plurality of through-holes (41), when present, is arranged at a distance comprised between 5-9 mm from the longitudinal development axis (24) of the first tubular element (2, 202), preferably 6.5 - 8 mm and also depends on the angle between the longitudinal development axes, and/or
- the holes (41) of the first and/or second plurality of through-holes (41) have dimensions of di 1.1 - 2 mm, preferably 1.6 x 0.9, and/or
- the holes of the third plurality of though-holes (28) have dimensions of 0.6 - 0.9 mm, preferably 0.7 x 0.85, and preferably 0.8.

It is particularly preferable, especially in the case of the double access vascular access device, for the length of the first tubular element to be 3-10 cm, more preferably 4-12 cm, 8-12 cm or advantageously 4-6 cm, both because in this way it is possible to implant it between a vein and an artery, in particular of an arm or a leg of a patient, and because in artificial conduits of this length it is possible for the same anatomic structure naturally present internally of the veins and the arteries to be recreated on the internal structure of the conduit. This significantly reduces the risk of blood clots and blocking of the first conduit (22, 222). Further, a length of the first tubular element (2, 202) of at least 4 cm, preferably 5-6 cm, ensures that the blood collected during dialysis does not contain blood just returned during dialysis. In a case where the vascular access device for haemodialysis is of the double access type, and is to be used to obtain an artificial arteriovenous fistula, the length is preferably at least 8-12 cm, which obviously depends on the patient's physical structure. Additionally, it is highly preferable for the device, or at least the inner walls of the first, second and third conduits (22, 222, 33, 233, 333), to be made of metal or biocompatible plastic materials. In the second case the device can be made by moulding or 3D printing. The preferred materials for making the device, especially if double access, are silicones, polymethyloxanes PDMSs, PTFE preferably ePTFE, DACRON, preferably silicones and/or polymethyloxanes and ePTFE, more preferably ePTFE, as they have a low propensity to thrombogenesis and enable a better endothelialisation of the relative first conduit (22, 220) which prevents the access device from becoming occluded; the risk of rejection is also reduced.

A vascular access device (1) for haemodialysis according to the invention is preferred further comprising a first valve (50) connected to the second conduit (33, 233) and configured so as, in a relative closed configuration, to hydraulically close the second conduit (33, 233) and, in a relative open configuration, to hydraulically open the second conduit (33, 233). In particular, in the case of a double access vascular access device, which therefore comprises the relative third tubular element (303), it is advantageous for it to also comprise a second valve (50) connected to the third conduit (333) and configured so as, in a relative closed configuration, to hydraulically close the third conduit (333) and so as, in a relative open configuration, to hydraulically open the third conduit (333). The presence of the first valve (50), in particular, in the case of a single access vascular access device, and of the first and second valve 50, in the case of a double access, advantageously enable limiting the blood losses once the haemodialysis has ended, improving the tolerability thereof by the patient. The valve (50) advantageously stops the blood flow as soon as the cannula of the single-use device (102) of collecting and/or injecting blood is extracted from the first conduit or from the second conduit at the end of dialysis. In this way it is possible to minimise blood loss from the patient subjected to dialysis, in particular when the patient is medicated with a fistula pressure pad on the vascular access comprising silver as described in patent documents EP2296717 and ITBO20080303, marketed by EMODIAL S.r.l.

The first and/or second valve (50) is preferably arranged, respectively, in the second conduit (3, 33) and/or in the third conduit (333) distally of the second end of the conduits. This enables using the vascular access healing device by inserting it at the vascular access point of the body and in the second conduit and/or in the third conduit to heal the valve access about the cylinder of the healing device as mentioned in the foregoing.

The first and/or the second valve (50) is advantageously configured to enable entry into the relative conduit (3, 233, 33) through the relative second end (21, 232) of the relative tubular element (2, 203, 303) of the first end (108) of the medical cannula (103) of the single-use device (102) of collecting and/or injecting.

In alternative preferred embodiments of the vascular access device for haemodialysis with single and double access, the first valve (50) is arranged between second end (21, 232) of the second tubular element (2, 203) and the first plate (4), wherein the first plate (4) comprises, at the relative first face, first engaging means (41) engaged with the second end (21, 232) of the second tubular element (203) and/or, when the third tubular element (303) is present, the second plate (304) and the second valve (50), the second valve (50) is arranged between the second end (332) of the third tubular element (303) and the second plate (304), the second plate (304) comprising, at the relative first face, second engaging means (41) for engaging in the second end (21, 232) of the third tubular element (303) (See figures 9A-10F).

In preferred embodiments of the vascular access device for haemodialysis, both single and double access, the first valve (50) is arranged on the second face of the first plate (4), opposite the relative first face and comprises third engaging means (54) for friction engagement with fourth engaging means (55) predisposed in the second end (21, 232) of the second tubular element (203) and/or in the first plate (4), and, when the third tubular element (303), the second plate (304) and the second valve (50) are present, the second valve (50) is arranged on the second face of the second plate (304), opposite the relative first face and comprises fifth engaging means (54) for friction engagement with sixth engaging means (55) predisposed in the second end (332) of the third tubular element (303) and/or in the second plate (304) (see figures 11A - 14E). The first engaging means (41), and/or the second engaging means (41), and/or the third engaging means (54), and/or the fifth engaging means (54), can comprise a relative plurality of engaging elements (41, 54) projecting from the first face by friction engagement with the second end (21, 232).

It is however preferable, as shown in the figures of the vascular access device for haemodialysis with single and double access, for the first valve (50) and/or the second valve (50), when present, to comprise a relative through-slot (56) which identifies two operating edges, wherein, in the relative closed configuration of the first and/or the second valve (50), the operating edges are in contact to prevent passage of fluids through the valve (50), and wherein the first and/or second valve (50) is elastically deformable to obtain the relative open configuration wherein the operating edges are moved away from one another to enable passage of a fluid through the slot (50) (see figures 18A-18N, in particular 18F). In a preferred aspect of the invention in the vascular access device for haemodialysis, both with single and double access, the first valve (50) and/or the second valve (50), when present, can comprise an elastically deformable element which, in the relative closed configuration, is not deformed and obstructs, respectively, the relative second conduit 33, 233 and/or the relative third conduit 333 and in a relative open configuration is compressed and does not obstruct the relative second conduit (33, 233) and/or the relative third conduit (333).

In alternative preferred embodiments of the vascular access device for haemodialysis with single and double access, the first valve (50) has a relative perimeter and is arranged in the second conduit (33, 233) at the first end (31, 231) of the second tubular element (3, 203) with a first portion (151) of the relative perimeter fixed to the second conduit (33, 233) and with a remaining second portion (152) of the relative perimeter which is free, and which in the relative closed configuration, faces and is in contact with the second conduit (3, 233) in order to hydraulically close the second conduit and/or (preferably and), when present, the third tubular element (303), the second plate (304) and the second valve (50), the last having a relative perimeter and being arranged in the third conduit (333) at the first end (332) of the third tubular element (303) with a first portion (151) of the relative perimeter fixed to the third conduit (333) and with a remaining second portion (152) of the relative perimeter which is free, and which in the relative closed configuration, faces and is in contact with the second conduit (233) in order to hydraulically close the second conduit (233); and wherein the first and/or second valve (50) is elastically deformable to obtain the relative open configuration wherein the relative second portion of the relative perimeter is not in contact with respectively the second conduit (3, 233) and/or with the third conduit (333) to hydraulically open, respectively the the second conduit (3, 233) and/or with the third conduit (333) (see figures 19A-19M in particular 19F and 19G).

In a particularly preferred aspect of the invention the vascular access device for haemodialysis of the double access invention, which thus comprises the third tubular element (303), further comprises: a first and a second contact surface (401, 402) (See figure 18D, 18N, 19E and 19N) which originate respectively from the first and second end (221, 223) of the first tubular element (202), wherein each of the first and second contact surface (401, 402) is configured to partially face a transversal section of an outer wall of blood vessel (A, V) in which a surgical incision has been made, completely surrounding the surgical incision, in order to facilitate the fixing, respectively, of the first and second end (221, 223) of the first tubular element (202) to the wall with the aim of hydraulically connecting the relative surgical incision to the first conduit (222). In this case, in the above-mentioned surgical method, the step of predisposing the vascular access device (1) for haemodialysis includes the double access vascular access device (1) has the first and second contact surface (401, 402) and the steps of fixing the first or second end (231, 232) of the first tubular element (202) to the vein and to the artery can advantageously comprise the positioning of the first and second contact surface (401, 402) at a transversal section, respectively of the vein and the artery facing the relative transversal sections and completely surrounding the relative surgical incision. Further, in this case, according to a particularly preferred aspect of the invention, each of the first and second contact surface (401, 402) comprises a fourth plurality of through-holes (48) (See figure 19E and 1M) predisposed to surround the surgical incision to allow the fixing by means of corresponding suture stitches passing through the relative plurality of through-holes of the first and second contact surface (401, 402) (See figures 18D, 18N, 19E and 19N) and through the wall of the blood vessel (A, V). Alternatively, it is preferable for each of the first and second contact surface (401, 402) (See figures 18D, 18N, 19E and 19N) to be continuous surfaces, smooth and/or knurled, to facilitate the fixing thereof using surgical glue to the wall of the blood vessel (A, V). In this case, in the above-mentioned surgical method, and the step of fixing the first or second end (231, 232) of the first tubular element (202) to the vein and to the artery can advantageously include gluing, using surgical glue, the first and second contact surface (401, 402), respectively, to the transversal section, respectively of the vein and the artery while the first and second contact surface (401, 402) face them and completely surround the relative surgical incision.

With the aim of maximising the collection and injection velocity of blood during haemodialysis a particular preference is for both sterile packages and a collection and/or injection system (1000) for haemodialysis according to the invention, wherein the first conduit (22) and the second conduit (33) have a relative minimum internal diameter and the cannula has a relative external diameter that is smaller by 0.2 - 0.3 mm than the smallest between the minimum internal diameters of the first conduit (22) and the second conduit (33). This enables, in fact, collection and/or injection of a greater blood flow.

For the purpose of maintaining sterility it is preferable that, in both the sterile package and in the collection and/or injection system for haemodialysis of the invention, the access element has a relative connection end which is: distal to the first end (108) of the medical cannula (103) and is engageable with a needle-free syringe, wherein the access element further comprises an elastically deformable element arranged in the access channel (181), and wherein the access channel (181) and the elastically deformable element are configured and mutually arranged in such a way that, when the connection end is disengaged, the elastically deformable element is not deformed and obstructs the access channel (181), in order to prevent a passage of liquids and aerosols, in such a way that, when the connection end is engaged with the needle-free syringe, the elastically deformable element is compressed by the needle-free syringe and de-obstructs the access channel (181) in order to hydraulically connect the syringe to the first end (108) of the medical cannula (103). In the package and the system the access element enables insertion of any anticoagulants into the access device, once implanted while the closing means (180) can be constituted by or can comprise a cap or lid, preferably friction-coupled, or a valve. The access element, when the first gripping means are present, can be arranged at the first gripping means (120) between the first gripping means (120) and the regulating and/or intercepting device (104) (see figure 5C). The first and/or the second gripping means can be constituted by a typical "butterfly" element that is usually applied to the medical cannulas.

To facilitate the insertion in the correct side of the first conduit (2, 202), the first end (108) of the medical cannula (103) through the second conduit (33, 203) and the first conduit (22, 222) or through the third conduit (333) and the first conduit (22, 222) of a vascular access device (1) the first conduit (22, 222) can comprise a first and/or a second protuberance (250) arranged respectively on the side opposite the second (33, 203) and third conduit (333), between, respectively, the second conduit (33, 202) and the first end (221) of the first tubular element (2, 202) and the third conduit (333), and the second end (223) of the first tubular element (202) and conformed so as to enable the first end (108) of the medical cannula (103) to pass over the protuberance and proceed respectively towards the first and second end (221, 223) of the first tubular element (202). To obtain the protuberances (250) it is possible to deform the first tubular element (202) from outside obtaining corresponding concavities (251) on the opposite side to the second and/or third tubular element (3, 202, 303) (See figures 17A-17C).

The system preferably also comprises a syringe containing a pharmaceutically injectable anticoagulant liquid composition, and more preferably packaged in a syringe, advantageously a needle-free syringe.

It is understood that the foregoing is described by way of example, and that any variants of a practical-applicational nature are taken to fall within the protective scope of the invention as described in the foregoing and as claimed in the following. The invention is defined by the appended claims.

## Claims

1. A vascular access device for haemodialysis permanently implantable in a patient having an arteriovenous fistula, the vascular access device being made of a biocompatible and sterilisable material, and comprising:
- a relative first tubular element (2, 202) having a first and a second relative end (21, 221, 223), which are longitudinal, the first tubular element (2, 202) defining a first conduit (22) which extends from the first to the second end (21, 221, 223);
- a relative second tubular element (3, 303) having a first and a second relative end (31, 32, 231, 232) which are longitudinal, and defining a second conduit (33, 233) which extends from the relative first to the second end (31, 32, 231, 232); wherein the first end (31, 231) of the second tubular element (3, 303) is fixed to the first tubular element (2, 202) in an intermediate position between the first and the second end (21, 221, 223) of the first tubular element (2, 202), so that the second conduit (33, 233) is in fluid communication with the first conduit (22, 222) and so that the second end (32, 232) of the second tubular element (3, 303) is on a first side with respect to the first tubular element (2, 202); and
- first fixing means (4) comprising a first plurality of through-holes (41) and being fixed to the first tubular element (2, 202) and/or second tubular element (3, 303) the first plurality of through-holes (41) being arranged on the first side, with respect to the first tubular element (2, 202), and at the second end (32, 232) of the second tubular element (3, 303), wherein said vascular access device (1) is conformed and dimensioned to be implantable in an arm of a patient having an arteriovenous fistula for haemodialysis with the first conduit (22, 222) interposed in series to the arteriovenous fistula, with respect to the blood flow therein, between a first portion (51) and a second portion (52) of the arteriovenous fistula, the first end (21, 221, 223) and the second end (21, 221, 223) of the first tubular element (2, 202) being fixed, respectively to the first portion (51) and to the second portion (52) of the arteriovenous fistula, at a relative end of the relative portion (51, 52) and the second end (32, 232) of the second tubular element (3, 303) and with the first plurality of through-holes (41) arranged subcutaneously (7) at an implant skin region (7) in order to enable fixing of the first plurality of through-holes (41) to the implant skin region (7) by means of a first plurality of suture stitches (60), and a consequent fixing of the second end (32, 232) of the second conduit (33, 233) to the implant skin region (7).

2. A vascular access device for haemodialysis of claim 1, wherein the first end (231) of the second tubular element (203) is fixed to the first tubular element (202) in proximity of the first end (221) of the first tubular element (202), in such a way that the second conduit (233) is in fluid communication with the first conduit (222) and so that the second end (232) of the second tubular element (203) is on a first side with respect to the first tubular element (202);
wherein the vascular access device further comprises:
- a relative third tubular element (303) having a first and a second relative end (331, 332) which are longitudinal, third tubular element (303) defining a third conduit (333) which extends from the relative first to the second end (331, 332); wherein the first end (331) of the third tubular element (303) is fixed to the first tubular element (202) in proximity of the second end (223) of the first tubular element (202), in such a way that the third conduit (333) is in fluid communication with the first conduit (222) and with the second conduit (33, 233) and so that the second end (332) of the third tubular element (303) is on the first side with respect to the first tubular element (202); and
- first fixing means (4) comprising a first plurality of through-holes (41) and being fixed to the first tubular element (202) and/or second tubular element (203), the first plurality of through-holes (41) being arranged on the first side, with respect to the first tubular element (202), and at the second end (32, 232) of the second tubular element (203),
- second fixing means (304) comprising a second plurality of through-holes (41) and being fixed to the first tubular element (202) and/or third tubular element (303), the second plurality of through-holes (41) being arranged on the first side, with respect to the first tubular element (202), and at the second end (332) of the third tubular element (303),
wherein the vascular access device (1) is conformed and dimensioned to be implanted in a patient in:
- a first position which is at an artery (A), at a vein (V), or at a pre-existing arteriovenous fistula (F) the first conduit being interposed in series with respect to the blood flow, respectively in the artery (A), in the vein (V) or in the pre-existing arteriovenous fistula (F) between a first portion (51) and a second portion (52), respectively, of the artery (A), vein (V), or pre-existing arteriovenous fistula (F), with the first end and the second end (21, 221, 223) of the first tubular element (202) fixed respectively to the first portion (51) and second portion (52), and/or
- in a second position, between the artery (A) and the vein (V), with the relative first or second end (31, 32, 231, 232) of the first tubular element (202) fixed to the artery (A) and with the remaining one of the first or second relative end (31, 32, 231, 232) of the first tubular element (202), which is not fixed to the artery (A), fixed to the vein (V) in such a way that the first conduit (222) hydraulically connects the artery (A) to the vein (V) to constitute an artificial arteriovenous fistula (FA),
in which, in the first and second position, the second end (32, 232) of the second tubular element (203) and the second end (332) of the third tubular element (303), the first and second plurality of through-holes (41) are arranged subcutaneously in an implant skin region (7) for implant in order to enable fixing of the first and second plurality of through-holes (41) to the implant skin region (7) by means of a corresponding first and a corresponding second plurality of suture stitches (60), and the consequent fixing, respectively, of the second end (32, 232) of the second conduit (233) and the second end (33, 332) of the third tubular element (303) to the implant skin region (7).

3. The vascular access device for haemodialysis of claim 1 or 2, wherein the first fixing means (4) originate from the second end (32, 232) of the second tubular element (3, 203) and/or wherein, when present, the second fixing means (304) originate from the second end (32, 332) of the third tubular element (3, 303); wherein the first fixing means (4) comprise: a plate (4) having a relative main through-hole (44), wherein the first plurality of through-holes (41) is arranged peripherally with respect to the main through-hole (44), wherein the plate (4) is fixed at a relative first face to the second end (32, 232) of the second tubular element (3, 203) in such a way that the second conduit (33, 233) is accessible from the relative main through-hole (44); and/or wherein the second fixing means, when present, comprise a second plate (304) having a relative main through-hole, wherein the second plurality of through-holes (41) is arranged peripherally with respect to the relative main through-hole wherein the second further plate (304) is fixed at a relative first face to the second end (332) of the third tubular element (303) so that the third conduit (333) is accessible from the relative main through-hole (44).

4. The vascular access device for haemodialysis of the preceding claim, wherein the plate (4) is fixed to the second tubular element (3, 203) by a relative second face to the second end (45) opposite the relative first face and arranged along a plane that is parallel to the longitudinal development axis (24) of the first tubular element (2, 202) and/or wherein, when present, the second plate (304) is fixed to the third tubular element (303) by a relative second face (45) opposite the relative first face and arranged along a plane that is parallel to the longitudinal development axis (24, 224) of the first tubular element (2, 202).

5. The vascular access device for haemodialysis of any one of claims from 1 to 4, further comprising a first valve (50) connected to the second conduit (33, 233) and configured so as, in a relative closed configuration, to hydraulically close the second conduit (33, 233) and so as, in a relative open configuration, to hydraulically open the second conduit (33, 233) and/or, when the relative third tubular element (303) is present, and a second valve (50) connected to the third conduit (333) and configured so as, in a relative closed configuration, to hydraulically close the third conduit (333) and so as, in a relative open configuration, to hydraulically open the third conduit (333).

6. The vascular access device for haemodialysis of claim 5, wherein the first valve (50) and/or the second valve (50) comprises a relative through-slot (56) which identifies two operating edges, wherein, in the relative closed configuration of the first and/or the second valve (50), the operating edges are in contact to prevent passage of fluids through the valve (50), and wherein the first and/or second valve (50) is elastically deformable to obtain the relative open configuration wherein the operating edges are moved away from one another to enable passage of a fluid through the slot (50).

7. The vascular access device for haemodialysis of claim 5, wherein the first valve (50) has a relative perimeter and is arranged in the second conduit (33, 233) at the first end (31, 231) of the second tubular element (203) with a first portion (151) of the relative perimeter fixed to the second conduit (33, 233) and with a remaining second portion (152) of the relative perimeter which is free, and which, in the relative closed configuration, faces and is in contact with the second conduit (33, 233) in order to hydraulically close the second conduit (33, 233) and/or wherein, when present, the second valve (50) has a relative perimeter and is arranged in the third conduit (333) at the first end (332) of the third tubular element (303) with a first portion (151) of the relative perimeter fixed to the third conduit (333) and with a remaining second portion (152) of the relative perimeter which is free, and which in the relative closed configuration, faces and is in contact with the second conduit (33, 233) in order to hydraulically close the second conduit (33, 233); and wherein the first and/or second valve (50) is elastically deformable to obtain the relative open configuration wherein the relative second portion of the relative perimeter is not in contact with, respectively, the second conduit (3, 233) and/or with the third conduit (333) in order to hydraulically open, respectively, the second conduit (3, 233) and/or the third conduit (333).

8. The vascular access device for haemodialysis of any one of claims from 2 to 7, further comprising: a first and a second contact surface (401, 402) which originate respectively from the first and second end (221, 223) of the first tubular element (202), wherein each of the first and second contact surface (401, 402) is configured to partially face a transversal section of an outer wall of blood vessel (A, V) in which a surgical incision has been made completely surrounding the surgical incision, in order to facilitate the fixing, respectively, of the first and second end (221, 223) of the first tubular element (202) to the wall with the aim of hydraulically connecting the relative surgical incision to the first conduit (222).

9. The vascular access device for haemodialysis of the preceding claim, wherein each of the first and second contact surface (401, 402) comprise a fourth plurality of through-holes (48) predisposed to surround the surgical incision to allow the fixing by means of corresponding suture stitches through the relative plurality of through-holes of the first and second contact surface (401, 402) and through the wall of the blood vessel (A, V).

10. A collection and/or injection system (1000) for haemodialysis, comprising:
- a vascular access device for haemodialysis of any one of claims from 1 to 9 and at least a single-use device (102) of collecting and/or injecting blood in an arteriovenous fistula (F), a vein (V) or an artery (A) constituted by:
- a medical cannula (103) for cannulation having a relative first end (108) and a relative second end (109) wherein the first end (108) is free and cannulable in a pre-existing arteriovenous fistula (F), an artery (A), or a vein (V) of a patient through the second conduit (33, 203) and the first conduit (22, 222) or through the third conduit (333) and the first conduit (22, 222) of a vascular access device (1);
- a flow regulating and/or intercepting device (104) predisposed to regulate and/or interrupt a flow of a liquid in the medical cannula (103);
- a connecting element (106), fixed to the second end (109) of the medical cannula (108) to enable hydraulic connection to an inlet tube of blood or to an outlet tube of blood of a haemodialysis apparatus;
- optionally an access element arranged between the first end (108) of the cannula and the regulating and/or intercepting device (104) and defining an access channel (181) hydraulically connected to the first end (108) of the cannula; and reversible closing means (180) for reversibly closing the access channel (181) at a distal end of the access element in order to enable injection of a liquid into the access channel (181) and to cause the liquid to flow towards the first end (108) of the medical cannula (103); and
- optionally a first gripping element (120), arranged between the first end (108) and/or the regulating and/or intercepting device (104), and/or a second gripping element (130), arranged proximally to the connecting element (106);
- wherein the collection and/or injection system (1000) comprises optionally at least one healing device (500) of a vascular access, comprising:
- a relative insertion portion (502) which is insertable internally of a vascular access and which comprises: a cylinder (505) having a relative longitudinal development axis: a first and a second relative end which are longitudinal; and a distally tapered tip (503) fixed to the first end of the cylinder (505); wherein the insertion portion is insertable in the second conduit (33, 233) or in the third conduit (333) of a vascular access device (1) for haemodialysis according to any one claim from 5 to 9 from the relative second end (32, 232, 332) so as to occlude exclusively a portion of the second conduit (33, 233) or the third conduit (333) comprised between, respectively, the first valve (50) and the first plate (4) or between the second valve (50) and the second plate (304);
- a relative connection portion (504) fixed to the second end of the cylinder (505); and
- a relative first and a relative second gripping tab (501), fixed to the connection portion (504) on opposite sides thereof and on opposite sides with respect to the cylinder axis and with possibility of relative movement with respect to the connection portion (504) in order to assume: a first relative configuration in which the first and the second gripping tab (501) are substantially coplanar; and a second configuration in which the tabs are opposite one another, wherein in both the first and the second configuration the first and the second gripping tab (501) are not insertable in the second conduit (33, 233) or third conduit (333), wherein, optionally the relative cylinder is inclined with respect to the connection portion by an angle (λ) identical to the angle (β) with which the second tubular element (3, 203) or the third tubular element (303) is inclined with respect, respectively, to the first plate (4) or the second plate (304).

## Patentansprüche

1. Vaskuläre Zugangsvorrichtung für die Hämodialyse, die dauerhaft in einen Patienten mit einer arteriovenösen Fistel implantiert werden kann, wobei die vaskuläre Zugangsvorrichtung aus einem biokompatiblen und sterilisierbaren Material hergestellt ist und umfasst:
- ein entsprechendes erstes röhrenförmiges Element (2, 202) mit einem ersten und einem zweiten entsprechenden Ende (21, 221, 223), die in Längsrichtung verlaufen, wobei das erste röhrenförmige Element (2, 202) eine erste Leitung (22) definiert, die sich vom ersten zum zweiten Ende (21, 221, 223) erstreckt;
- ein entsprechendes zweites röhrenförmiges Element (3, 303), das ein erstes und ein zweites entsprechendes Ende (31, 32, 231, 232) aufweist, die in Längsrichtung verlaufen, und das eine zweite Leitung (33, 233) definiert, die sich von dem entsprechenden ersten zu dem zweiten Ende (31, 32, 231, 232) erstreckt; wobei das erste Ende (31, 231) des zweiten rohrförmigen Elements (3, 303) an dem ersten rohrförmigen Element (2, 202) in einer Zwischenposition zwischen dem ersten und dem zweiten Ende (21, 221, 223) des ersten rohrförmigen Elements (2, 202) befestigt ist, so dass die zweite Leitung (33, 233) in Fluidverbindung mit der ersten Leitung (22, 222) steht und so dass das zweite Ende (32, 232) des zweiten rohrförmigen Elements (3, 303) sich auf einer ersten Seite in Bezug auf das erste rohrförmige Element (2, 202) befindet; und
- ein erstes Befestigungsmittel (4), das eine erste Mehrzahl von Durchgangslöchern (41) umfasst und an dem ersten röhrenförmigen Element (2, 202) und/oder dem zweiten röhrenförmigen Element (3, 303) befestigt ist, wobei die erste Mehrzahl von Durchgangslöchern (41) auf der ersten Seite in Bezug auf das erste röhrenförmige Element (2, 202) und an dem zweiten Ende (32, 232) des zweiten röhrenförmigen Elements (3, 303) angeordnet ist, wobei die Gefäßzugangsvorrichtung (1) so gestaltet und dimensioniert ist, dass sie in einen Arm eines Patienten mit einer arteriovenösen Fistel für die Hämodialyse implantiert werden kann, wobei die erste Leitung (22, 222) in Bezug auf den Blutfluss in der arteriovenösen Fistel in Reihe zwischen einem ersten Abschnitt (51) und einem zweiten Abschnitt (52) der arteriovenösen Fistel angeordnet ist, das erste Ende (21, 221, 223) und das zweite Ende (21, 221, 223) des ersten röhrenförmigen Elements (2, 202) jeweils an dem ersten Abschnitt (51) und dem zweiten Abschnitt (52) der arteriovenösen Fistel an einem relativen Ende des relativen Abschnitts (51, 52) und dem zweiten Ende (32, 232) des zweiten röhrenförmigen Elements (3, 303) und mit der ersten Mehrzahl von Durchgangslöchern (41), die subkutan (7) an einem Implantathautbereich (7) angeordnet sind, um eine Fixierung der ersten Mehrzahl von Durchgangslöchern (41) an dem Implantathautbereich (7) mittels einer ersten Mehrzahl von Nahtstichen (60) und eine anschließende Fixierung des zweiten Endes (32, 232) der zweiten Leitung (33, 233) an dem Implantathautbereich (7) zu ermöglichen.

2. Vaskuläre Zugangsvorrichtung für die Hämodialyse nach Anspruch 1, wobei das erste Ende (231) des zweiten röhrenförmigen Elements (203) an dem ersten röhrenförmigen Element (202) in der Nähe des ersten Endes (221) des ersten röhrenförmigen Elements (202) so befestigt ist, dass die zweite Leitung (233) in Flüssigkeitsverbindung mit der ersten Leitung (222) steht und dass das zweite Ende (232) des zweiten röhrenförmigen Elements (203) auf einer ersten Seite in Bezug auf das erste röhrenförmige Element (202) liegt;
wobei die vaskuläre Zugangsvorrichtung ferner umfasst:
- ein relatives drittes röhrenförmiges Element (303) mit einem ersten und einem zweiten relativen Ende (331, 332), die in Längsrichtung verlaufen, wobei das dritte röhrenförmige Element (303) eine dritte Leitung (333) definiert, die sich von dem relativen ersten zu dem zweiten Ende (331, 332) erstreckt; wobei das erste Ende (331) des dritten röhrenförmigen Elements (303) an dem ersten röhrenförmigen Element (202) in der Nähe des zweiten Endes (223) des ersten röhrenförmigen Elements (202) derart befestigt ist, dass die dritte Leitung (333) in Fluidverbindung mit der ersten Leitung (222) und mit der zweiten Leitung (33, 233) steht und dass das zweite Ende (332) des dritten röhrenförmigen Elements (303) sich auf der ersten Seite in Bezug auf das erste röhrenförmige Element (202) befindet; und
- ein erstes Befestigungsmittel (4), das eine erste Vielzahl von Durchgangslöchern (41) umfasst und an dem ersten rohrförmigen Element (202) und/oder dem zweiten rohrförmigen Element (203) befestigt ist, wobei die erste Vielzahl von Durchgangslöchern (41) auf der ersten Seite in Bezug auf das erste rohrförmige Element (202) und an dem zweiten Ende (32, 232) des zweiten rohrförmigen Elements (203) angeordnet ist,
- ein zweites Befestigungsmittel (304), das eine zweite Vielzahl von Durchgangslöchern (41) umfasst und an dem ersten rohrförmigen Element (202) und/oder dem dritten rohrförmigen Element (303) befestigt ist, wobei die zweite Vielzahl von Durchgangslöchern (41) auf der ersten Seite in Bezug auf das erste rohrförmige Element (202) und am zweiten Ende (332) des dritten rohrförmigen Elements (303) angeordnet ist,
wobei die vaskuläre Zugangsvorrichtung (1) so geformt und dimensioniert ist, dass sie in einen Patienten implantiert werden kann in:
- einer ersten Position, die sich an einer Arterie (A), an einer Vene (V) oder an einer bereits existierenden arteriovenösen Fistel (F) befindet, wobei die erste Leitung in Bezug auf den Blutfluss in der Arterie (A), in der Vene (V) oder in der bereits existierenden arteriovenösen Fistel (F) zwischen einem ersten Abschnitt (51) und einem zweiten Abschnitt (52) in Reihe geschaltet ist, zwischen einem ersten Abschnitt (51) und einem zweiten Abschnitt (52) der Arterie (A), der Vene (V) oder der bereits bestehenden arteriovenösen Fistel (F), wobei das erste Ende und das zweite Ende (21, 221, 223) des ersten rohrförmigen Elements (202) an dem ersten Abschnitt (51) bzw. dem zweiten Abschnitt (52) befestigt sind, und/oder
- in einer zweiten Position zwischen der Arterie (A) und der Vene (V), wobei das entsprechende erste oder zweite Ende (31, 32, 231, 232) des ersten rohrförmigen Elements (202) an der Arterie (A) befestigt ist und das verbleibende des ersten oder zweiten entsprechenden Endes (31, 32, 231, 232) des ersten rohrförmigen Elements (202), das nicht an der Arterie (A) befestigt ist, an der Vene (V) befestigt ist, so dass die erste Leitung (222) die Arterie (A) hydraulisch mit der Vene (V) verbindet, um eine künstliche arteriovenöse Fistel (FA) zu bilden,
in der in der ersten und zweiten Position das zweite Ende (32, 232) des zweiten röhrenförmigen Elements (203) und das zweite Ende (332) des dritten röhrenförmigen Elements (303), die erste und zweite Vielzahl von Durchgangslöchern (41) subkutan in einem zu implantierenden Hautbereich (7) angeordnet sind, um die Fixierung der ersten und zweiten Vielzahl von Durchgangslöchern (41) an dem zu implantierenden Hautbereich (7) mittels einer entsprechenden ersten und einer entsprechenden zweiten Vielzahl von Nahtstichen (60) zu ermöglichen, und die anschließende Befestigung des zweiten Endes (32, 232) des zweiten Kanals (233) bzw. des zweiten Endes (33, 332) des dritten rohrförmigen Elements (303) an der Implantathautregion (7).

3. Vaskuläre Zugangsvorrichtung für die Hämodialyse nach Anspruch 1 oder 2, wobei die ersten Befestigungsmittel (4) von dem zweiten Ende (32, 232) des zweiten rohrförmigen Elements (3, 203) ausgehen und/oder wobei, falls vorhanden, die zweiten Befestigungsmittel (304) von dem zweiten Ende (32, 332) des dritten rohrförmigen Elements (3, 303) ausgehen; wobei die ersten Befestigungsmittel (4) umfassen: eine Platte (4) mit einem relativen Hauptdurchgangsloch (44), wobei die erste Mehrzahl von Durchgangslöchern (41) in Bezug auf das Hauptdurchgangsloch (44) am Umfang angeordnet ist, wobei die Platte (4) an einer relativen ersten Fläche an dem zweiten Ende (32, 232) des zweiten rohrförmigen Elements (3, 203) derart befestigt ist, dass die zweite Leitung (33, 233) von dem relativen Hauptdurchgangsloch (44) aus zugänglich ist; und/oder wobei die zweiten Befestigungsmittel, falls vorhanden, eine zweite Platte (304) mit einem relativen Hauptdurchgangsloch umfassen, wobei die zweite Vielzahl von Durchgangslöchern (41) in Bezug auf das relative Hauptdurchgangsloch am Umfang angeordnet ist, wobei die zweite weitere Platte (304) an einer relativen ersten Fläche an dem zweiten Ende (332) des dritten rohrförmigen Elements (303) befestigt ist, so dass die dritte Leitung (333) von dem relativen Hauptdurchgangsloch (44) aus zugänglich ist.

4. Die vaskuläre Zugangsvorrichtung für die Hämodialyse nach dem vorhergehenden Anspruch, wobei die Platte (4) an dem zweiten rohrförmigen Element (3, 203) durch eine relative zweite Fläche an dem zweiten Ende (45) gegenüber der relativen ersten Fläche befestigt ist und entlang einer Ebene angeordnet ist, die parallel zu der Längsentwicklungsachse (24) des ersten rohrförmigen Elements (2, 202) angeordnet ist und/oder wobei, wenn vorhanden, die zweite Platte (304) an dem dritten rohrförmigen Element (303) durch eine relative zweite Fläche (45) gegenüber der relativen ersten Fläche befestigt ist und entlang einer Ebene angeordnet ist, die parallel zu der Längsentwicklungsachse (24, 224) des ersten rohrförmigen Elements (2, 202) ist.

5. Die vaskuläre Zugangsvorrichtung für die Hämodialyse nach einem der Ansprüche 1 bis 4, die ferner ein erstes Ventil (50) umfasst, das mit der zweiten Leitung (33, 233) verbunden ist und so konfiguriert ist, dass es in einer relativ geschlossenen Konfiguration die zweite Leitung (33, 233) hydraulisch schließt und in einer relativ offenen Konfiguration die zweite Leitung (33, 233) hydraulisch zu schließen und in einer relativ offenen Konfiguration die zweite Leitung (33, 233) hydraulisch zu öffnen und/oder, wenn das entsprechende dritte rohrförmige Element (303) vorhanden ist, und ein zweites Ventil (50), das mit der dritten Leitung (333) verbunden und so konfiguriert ist, dass es in einer relativ geschlossenen Konfiguration die dritte Leitung (333) hydraulisch schließt und in einer relativ offenen Konfiguration die dritte Leitung (333) hydraulisch öffnet.

6. Vaskuläre Zugangsvorrichtung für die Hämodialyse nach Anspruch 5, wobei das erste Ventil (50) und/oder das zweite Ventil (50) einen entsprechenden Durchgangsschlitz (56) aufweist, der zwei Arbeitskanten identifiziert, wobei in der relativ geschlossenen Konfiguration des ersten und/oder des zweiten Ventils (50) die Arbeitskanten in Kontakt sind, um den Durchgang von Fluiden durch das Ventil (50) zu verhindern, und wobei das erste und/oder das zweite Ventil (50) elastisch verformbar ist, um die relativ offene Konfiguration zu erhalten, in der die Arbeitskanten voneinander weg bewegt werden, um den Durchgang eines Fluids durch den Schlitz (50) zu ermöglichen.

7. Vaskuläre Zugangsvorrichtung für die Hämodialyse nach Anspruch 5, wobei das erste Ventil (50) einen relativen Umfang aufweist und in der zweiten Leitung (33, 233) am ersten Ende (31, 231) des zweiten rohrförmigen Elements (203) angeordnet ist, wobei ein erster Teil (151) des relativen Umfangs an der zweiten Leitung (33, 233) befestigt ist, und mit einem verbleibenden zweiten Teil (152) des relativen Umfangs, der frei ist und der in der relativ geschlossenen Konfiguration der zweiten Leitung (33, 233) zugewandt ist und mit ihr in Kontakt steht, um die zweite Leitung (33, 233) hydraulisch zu schließen, 233) zu schließen und/oder wobei, wenn vorhanden, das zweite Ventil (50) einen relativen Umfang hat und in der dritten Leitung (333) am ersten Ende (332) des dritten rohrförmigen Elements (303) angeordnet ist, wobei ein erster Teil (151) des relativen Umfangs an der dritten Leitung (333) befestigt ist und ein verbleibender zweiter Teil (152) des relativen Umfangs frei ist und in der relativ geschlossenen Konfiguration der zweiten Leitung (33, 233) zugewandt ist und mit ihr in Kontakt steht, um die zweite Leitung (33, 233) hydraulisch zu schließen; und wobei das erste und/oder zweite Ventil (50) elastisch verformbar ist, um die relative offene Konfiguration zu erhalten, in der der relative zweite Teil des relativen Umfangs nicht in Kontakt mit der zweiten Leitung (3, 233) und/oder mit der dritten Leitung (333) steht, um die zweite Leitung (3, 233) und/oder die dritte Leitung (333) hydraulisch zu öffnen.

8. Die vaskuläre Zugangsvorrichtung für die Hämodialyse nach einem der Ansprüche 2 bis 7, ferner umfassend: eine erste und eine zweite Kontaktfläche (401, 402), die vom ersten bzw. zweiten Ende (221, 223) des ersten rohrförmigen Elements (202) ausgehen, wobei sowohl die erste als auch die zweite Kontaktfläche (401, 402) so konfiguriert ist, dass sie teilweise einem Querschnitt einer Außenwand eines Blutgefäßes (A, V) zugewandt ist, in dem ein chirurgischer Einschnitt vorgenommen wurde, der den chirurgischen Einschnitt vollständig umgibt, um die Befestigung des ersten bzw. zweiten Endes (221, 223) des ersten rohrförmigen Elements (202) an der Wand zu erleichtern, um den entsprechenden chirurgischen Einschnitt hydraulisch mit der ersten Leitung (222) zu verbinden.

9. Die Gefäßzugangsvorrichtung für die Hämodialyse nach dem vorhergehenden Anspruch, wobei die erste und die zweite Kontaktfläche (401, 402) jeweils eine vierte Vielzahl von Durchgangslöchern (48) aufweisen, die so angeordnet sind, dass sie den chirurgischen Einschnitt umgeben, um die Fixierung mittels entsprechender Nahtstiche durch die jeweilige Vielzahl von Durchgangslöchern der ersten und zweiten Kontaktfläche (401, 402) und durch die Wand des Blutgefäßes (A, V) zu ermöglichen.

10. Ein Sammel- und/oder Injektionssystem (1000) für die Hämodialyse, umfassend:
- eine vaskuläre Zugangsvorrichtung für die Hämodialyse nach einem der Ansprüche 1 bis 9 und mindestens eine Einwegvorrichtung (102) zum Sammeln und/oder Injizieren von Blut in eine arteriovenöse Fistel (F), eine Vene (V) oder eine Arterie (A), gebildet durch:
- eine medizinische Kanüle (103) zur Kanülierung mit einem entsprechenden ersten Ende (108) und einem entsprechenden zweiten Ende (109), wobei das erste Ende (108) frei ist und in eine bereits bestehende arteriovenöse Fistel (F), eine Arterie (A) oder eine Vene (V) eines Patienten durch die zweite Leitung (33, 203) und die erste Leitung (22, 222) oder durch die dritte Leitung (333) und die erste Leitung (22, 222) einer Gefäßzugangsvorrichtung (1) kanüliert werden kann;
- eine Durchflussregulierungs- und/oder Unterbrechungsvorrichtung (104), die dazu vorgesehen ist, den Fluss einer Flüssigkeit in der medizinischen Kanüle (103) zu regulieren und/oder zu unterbrechen;
- ein Verbindungselement (106), das am zweiten Ende (109) der medizinischen Kanüle (108) befestigt ist, um eine hydraulische Verbindung mit einem Blutzufuhrschlauch oder einem Blutauslassschlauch eines Hämodialysegeräts zu ermöglichen;
- optional ein Zugangselement, das zwischen dem ersten Ende (108) der Kanüle und der Regulier- und/oder Abfangvorrichtung (104) angeordnet ist und einen Zugangskanal (181) definiert, der hydraulisch mit dem ersten Ende (108) der Kanüle verbunden ist; und reversible Verschlussmittel (180) zum reversiblen Verschließen des Zugangskanals (181) an einem distalen Ende des Zugangselements, um die Injektion einer Flüssigkeit in den Zugangskanal (181) zu ermöglichen und zu bewirken, dass die Flüssigkeit zum ersten Ende (108) der medizinischen Kanüle (103) fließt; und
- optional ein erstes Greifelement (120), das zwischen dem ersten Ende (108) und/oder der Regulierungs- und/oder Abfangvorrichtung (104) angeordnet ist, und/oder ein zweites Greifelement (130), das proximal zu dem Verbindungselement (106) angeordnet ist;
- wobei das Entnahme- und/oder Injektionssystem (1000) optional mindestens eine Heilungsvorrichtung (500) eines vaskulären Zugangs umfasst, umfassend:
- einen relativen Einführungsabschnitt (502), der in das Innere eines vaskulären Zugangs einführbar ist und der umfasst: einen Zylinder (505) mit einer relativen Längsentwicklungsachse: ein erstes und ein zweites relatives Ende, die in Längsrichtung verlaufen; und eine distal verjüngte Spitze (503), die an dem ersten Ende des Zylinders (505) befestigt ist; wobei der Einführungsabschnitt in die zweite Leitung (33, 233) oder in die dritte Leitung (333) einer vaskulären Zugangsvorrichtung (1) für die Hämodialyse gemäß einem der Ansprüche 5 bis 9 von dem relativen zweiten Ende (32, 232, 332) aus einführbar ist, um ausschließlich einen Abschnitt der zweiten Leitung (33, 233) oder der dritten Leitung (333) zu verschließen, der zwischen dem ersten Ventil (50) und der ersten Platte (4) bzw. zwischen dem zweiten Ventil (50) und der zweiten Platte (304) liegt;
- einen relativen Verbindungsteil (504), der am zweiten Ende des Zylinders (505) befestigt ist; und
- eine relative erste und eine relative zweite Greiflasche (501), die an dem Verbindungsabschnitt (504) auf gegenüberliegenden Seiten desselben und auf gegenüberliegenden Seiten in Bezug auf die Zylinderachse befestigt sind und mit der Möglichkeit einer relativen Bewegung in Bezug auf den Verbindungsabschnitt (504), um eine eine erste relative Konfiguration, in der die erste und die zweite Greiflasche (501) im Wesentlichen koplanar sind; und eine zweite Konfiguration, in der die Laschen einander gegenüberliegen, wobei sowohl in der ersten als auch in der zweiten Konfiguration die erste und die zweite Greiflasche (501) nicht in die zweite Leitung (33, 233) oder die dritte Leitung (333) einführbar sind, wobei, optional der relative Zylinder in Bezug auf den Verbindungsabschnitt um einen Winkel (λ) geneigt ist, der identisch mit dem Winkel (β) ist, mit dem das zweite rohrförmige Element (3, 203) oder das dritte rohrförmige Element (303) in Bezug auf die erste Platte (4) bzw. die zweite Platte (304) geneigt ist.

## Revendications

1. Dispositif d'accès vasculaire pour hémodialyse implantable en permanence chez un patient présentant une fistule artérioveineuse, le dispositif d'accès vasculaire étant constitué d'un matériau biocompatible et stérilisable, et comprenant :
- un premier élément tubulaire relatif (2, 202) ayant une première et une deuxième extrémité relative (21, 221, 223), qui sont longitudinales, le premier élément tubulaire (2, 202) définissant un premier conduit (22) qui s'étend de la première à la deuxième extrémité (21, 221, 223) ;
- un second élément tubulaire relatif (3, 303) ayant une première et une seconde extrémité relative (31, 32, 231, 232) qui sont longitudinales, et définissant un second conduit (33, 233) qui s'étend de la première à la seconde extrémité relative (31, 32, 231, 232) ; dans lequel la première extrémité (31, 231) du deuxième élément tubulaire (3, 303) est fixée au premier élément tubulaire (2, 202) dans une position intermédiaire entre la première et la deuxième extrémité (21, 221, 223) du premier élément tubulaire (2, 202), de sorte que le second conduit (33, 233) soit en communication fluidique avec le premier conduit (22, 222) et que la seconde extrémité (32, 232) du second élément tubulaire (3, 303) se trouve sur un premier côté par rapport au premier élément tubulaire (2, 202) ; et
- un premier moyen de fixation (4) comprenant une première série de trous traversants (41) et fixé au premier élément tubulaire (2, 202) et/ou au deuxième élément tubulaire (3, 303), la première série de trous traversants (41) étant disposée sur le premier côté, par rapport au premier élément tubulaire (2, 202), et à la deuxième extrémité (32, 232) du deuxième élément tubulaire (3, 303), dans lequel ledit dispositif d'accès vasculaire (1) est conforme et dimensionné pour être implanté dans le bras d'un patient ayant une fistule artérioveineuse pour l'hémodialyse avec le premier conduit (22, 222) interposé en série à la fistule artérioveineuse, par rapport au flux sanguin dans celle-ci, entre une première partie (51) et une deuxième partie (52) de la fistule artérioveineuse, la première extrémité (21, 221, 223) et la seconde extrémité (21, 221, 223) du premier élément tubulaire (2, 202) étant fixées respectivement à la première portion (51) et à la seconde portion (52) de la fistule artérioveineuse, à une extrémité relative de la portion relative (51, 52) et à la seconde extrémité (32, 232) du second élément tubulaire (3, 303) et avec la première pluralité de trous de passage (41) disposés en sous-cutané (7) au niveau d'une région cutanée de l'implant (7) afin de permettre la fixation de la première pluralité de trous de passage (41) à la région cutanée de l'implant (7) au moyen d'une première pluralité de points de suture (60), et une fixation conséquente de la seconde extrémité (32, 232) du second conduit (33, 233) à la région cutanée de l'implant (7).

2. Dispositif d'accès vasculaire pour l'hémodialyse de la revendication 1, dans lequel la première extrémité (231) du second élément tubulaire (203) est fixée au premier élément tubulaire (202) à proximité de la première extrémité (221) du premier élément tubulaire (202), de telle sorte que le second conduit (233) soit en communication fluidique avec le premier conduit (222) et que la seconde extrémité (232) du second élément tubulaire (203) soit du premier côté par rapport au premier élément tubulaire (202) ;
dans lequel le dispositif d'accès vasculaire comprend en outre
- un troisième élément tubulaire relatif (303) ayant une première et une deuxième extrémité relative (331, 332) qui sont longitudinales, le troisième élément tubulaire (303) définissant un troisième conduit (333) qui s'étend de la première à la deuxième extrémité relative (331, 332) ; dans lequel la première extrémité (331) du troisième élément tubulaire (303) est fixée au premier élément tubulaire (202) à proximité de la deuxième extrémité (223) du premier élément tubulaire (202), de telle sorte que le troisième conduit (333) soit en communication fluidique avec le premier conduit (222) et avec le deuxième conduit (33, 233) et que la deuxième extrémité (332) du troisième élément tubulaire (303) se trouve du premier côté par rapport au premier élément tubulaire (202) ; et
- premier moyen de fixation (4) comprenant une première pluralité de trous traversants (41) et fixé au premier élément tubulaire (202) et/ou au deuxième élément tubulaire (203), la première pluralité de trous traversants (41) étant disposée sur le premier côté, par rapport au premier élément tubulaire (202), et à la deuxième extrémité (32, 232) du deuxième élément tubulaire (203),
- deuxième moyen de fixation (304) comprenant une deuxième série de trous traversants (41) et fixé au premier élément tubulaire (202) et/ou au troisième élément tubulaire (303), la deuxième série de trous traversants (41) étant disposée sur le premier côté, par rapport au premier élément tubulaire (202), et à la deuxième extrémité (332) du troisième élément tubulaire (303),
dans lequel le dispositif d'accès vasculaire (1) est conformé et dimensionné pour être implanté dans un patient dans :
- une première position qui se situe au niveau d'une artère (A), d'une veine (V), ou d'une fistule artérioveineuse préexistante (F) le premier conduit étant interposé en série par rapport au flux sanguin, respectivement dans l'artère (A), dans la veine (V) ou dans la fistule artérioveineuse préexistante (F) entre une première portion (51) et une seconde portion (52), respectivement, de l'artère (A), de la veine (V) ou de la fistule artérioveineuse préexistante (F), la première extrémité et la seconde extrémité (21, 221, 223) du premier élément tubulaire (202) étant fixées respectivement à la première portion (51) et à la seconde portion (52), et/ou
- dans une deuxième position, entre l'artère (A) et la veine (V), la première ou la deuxième extrémité relative (31, 32, 231, 232) du premier élément tubulaire (202) étant fixée à l'artère (A) et l'autre de la première ou de la deuxième extrémité relative (31, 32, 231, 232) du premier élément tubulaire (202), qui n'est pas fixée à l'artère (A), fixée à la veine (V) de telle sorte que le premier conduit (222) relie hydrauliquement l'artère (A) à la veine (V) pour constituer une fistule artérioveineuse artificielle (FA),
dans laquelle, dans la première et la deuxième position, la deuxième extrémité (32, 232) du deuxième élément tubulaire (203) et la deuxième extrémité (332) du troisième élément tubulaire (303), la première et la deuxième pluralité de trous de passage (41) sont disposées sous-cutanément dans une région de la peau de l'implant (7) pour l'implantation afin de permettre la fixation de la première et de la deuxième pluralité de trous de passage (41) à la région de la peau de l'implant (7) au moyen d'une première et d'une deuxième pluralité correspondantes de points de suture (60), et la fixation consécutive, respectivement, de la deuxième extrémité (32, 232) du deuxième conduit (233) et de la deuxième extrémité (33, 332) du troisième élément tubulaire (303) à la région de la peau de l'implant (7).

3. Le dispositif d'accès vasculaire pour hémodialyse de la revendication 1 ou 2, dans lequel les premiers moyens de fixation (4) proviennent de la deuxième extrémité (32, 232) du deuxième élément tubulaire (3, 203) et/ou dans lequel, lorsqu'ils sont présents, les seconds moyens de fixation (304) proviennent de la deuxième extrémité (32, 332) du troisième élément tubulaire (3, 303) ; dans lequel les premiers moyens de fixation (4) comprennent : une plaque (4) ayant un trou de passage principal relatif (44), dans laquelle la première pluralité de trous de passage (41) est disposée en périphérie par rapport au trou de passage principal (44), dans laquelle la plaque (4) est fixée sur une première face relative à la deuxième extrémité (32, 232) du deuxième élément tubulaire (3, 203) de telle sorte que le deuxième conduit (33, 233) est accessible à partir du trou de passage principal relatif (44) ; et/ou dans lequel les seconds moyens de fixation, lorsqu'ils existent, comprennent une seconde plaque (304) ayant un trou de passage principal relatif, dans lequel la seconde pluralité de trous de passage (41) est disposée en périphérie par rapport au trou de passage principal relatif, dans lequel la seconde plaque supplémentaire (304) est fixée sur une première face relative à la seconde extrémité (332) du troisième élément tubulaire (303) de manière à ce que le troisième conduit (333) soit accessible à partir du trou de passage principal relatif (44).

4. Le dispositif d'accès vasculaire pour hémodialyse de la revendication précédente, dans lequel la plaque (4) est fixée au second élément tubulaire (3, 203) par une seconde face relative à la seconde extrémité (45) opposée à la première face relative et disposée le long d'un plan parallèle à l'axe de développement longitudinal (24) du premier élément tubulaire (2, 202) et/ou dans lequel, lorsqu'elle est présente, la plaque (4) est fixée à la seconde extrémité (45) opposée à la première face relative, 202) et/ou dans lequel, lorsqu'elle est présente, la seconde plaque (304) est fixée au troisième élément tubulaire (303) par une seconde face relative (45) opposée à la première face relative et disposée le long d'un plan parallèle à l'axe de développement longitudinal (24, 224) du premier élément tubulaire (2, 202).

5. Le dispositif d'accès vasculaire pour hémodialyse de l'une quelconque des revendications 1 à 4, comprenant en outre une première valve (50) connectée au second conduit (33, 233) et configurée de manière à, dans une configuration fermée relative, fermer hydrauliquement le second conduit (33, 233) et de manière à, dans une configuration ouverte relative, ouvrir hydrauliquement le second conduit (33, 233) et/ou, lorsque la troisième face relative du second conduit (33, 233) est ouverte, de manière à fermer hydrauliquement le second conduit (33, 233), 233) et/ou, lorsque le troisième élément tubulaire (303) est présent, et une deuxième vanne (50) connectée au troisième conduit (333) et configurée pour, dans une configuration de fermeture relative, fermer hydrauliquement le troisième conduit (333) et pour, dans une configuration d'ouverture relative, ouvrir hydrauliquement le troisième conduit (333).

6. Le dispositif d'accès vasculaire pour hémodialyse de la revendication 5, dans lequel la première valve (50) et/ou la seconde valve (50) comprend une fente de passage relative (56) qui identifie deux bords de fonctionnement, dans laquelle, dans la configuration fermée relative de la première et/ou de la seconde valve (50), les bords de fonctionnement sont en contact pour empêcher le passage de fluides à travers la valve (50), et dans laquelle la première et/ou la seconde valve (50) est élastiquement déformable pour obtenir la configuration ouverte relative dans laquelle les bords de fonctionnement sont éloignés l'un de l'autre pour permettre le passage d'un fluide à travers la fente (50).

7. Dispositif d'accès vasculaire pour hémodialyse de la revendication 5, dans lequel la première valve (50) a un périmètre relatif et est disposée dans le second conduit (33, 233) à la première extrémité (31, 231) du second élément tubulaire (203) avec une première partie (151) du périmètre relatif fixée au second conduit (33, 233) et avec une seconde portion (152) restante du périmètre relatif qui est libre et qui, dans la configuration fermée relative, fait face et est en contact avec le second conduit (33, 233) afin de fermer hydrauliquement le second conduit (33, 233) et/ou dans lequel, lorsqu'il est présent, le deuxième élément tubulaire (203) est fixé à la première extrémité (31, 231) du second conduit (33, 233), 233) et/ou dans lequel, lorsqu'elle est présente, la deuxième soupape (50) a un périmètre relatif et est disposée dans le troisième conduit (333) à la première extrémité (332) du troisième élément tubulaire (303) avec une première partie (151) du périmètre relatif fixée au troisième conduit (333) et avec une deuxième partie (152) restante du périmètre relatif qui est libre et qui, dans la configuration fermée relative, fait face et est en contact avec le deuxième conduit (33, 233) afin de fermer hydrauliquement le deuxième conduit (33, 233) ; et dans lequel la première et/ou la deuxième valve (50) est élastiquement déformable pour obtenir la configuration ouverte relative dans laquelle la deuxième partie relative du périmètre relatif n'est pas en contact avec, respectivement, le deuxième conduit (3, 233) et/ou le troisième conduit (333) afin d'ouvrir hydrauliquement, respectivement, le deuxième conduit (3, 233) et/ou le troisième conduit (333).

8. Le dispositif d'accès vasculaire pour hémodialyse de l'une quelconque des revendications 2 à 7, comprenant en outre : une première et une deuxième surface de contact (401, 402) qui proviennent respectivement de la première et de la deuxième extrémité (221, 223) du premier élément tubulaire (202), dans laquelle chacune de la première et de la deuxième surface de contact (401, 402) est configurée pour faire partiellement face à une section transversale d'une paroi externe d'un vaisseau sanguin (A, V) dans lequel une incision chirurgicale a été pratiquée, entourant complètement l'incision chirurgicale, afin de faciliter la fixation, respectivement, de la première et de la deuxième extrémité (221, 223) du premier élément tubulaire (202) à la paroi dans le but de connecter hydrauliquement l'incision chirurgicale relative au premier conduit (222).

9. Dispositif d'accès vasculaire pour hémodialyse de la revendication précédente, dans lequel la première et la deuxième surface de contact (401, 402) comprennent chacune une quatrième pluralité de trous de passage (48) prédisposés à entourer l'incision chirurgicale pour permettre la fixation au moyen de points de suture correspondants à travers la pluralité relative de trous de passage de la première et de la deuxième surface de contact (401, 402) et à travers la paroi du vaisseau sanguin (A, V).

10. Système de collecte et/ou d'injection (1000) pour l'hémodialyse, comprenant :
- un dispositif d'accès vasculaire pour hémodialyse de l'une quelconque des revendications de 1 à 9 et au moins un dispositif à usage unique (102) de prélèvement et/ou d'injection de sang dans une fistule artérioveineuse (F), une veine (V) ou une artère (A) constitué par :
- une canule médicale (103) pour la canulation ayant une première extrémité relative (108) et une deuxième extrémité relative (109) dans laquelle la première extrémité (108) est libre et canulable dans une fistule artérioveineuse préexistante (F), une artère (A), ou une veine (V) d'un patient à travers le deuxième conduit (33, 203) et le premier conduit (22, 222) ou à travers le troisième conduit (333) et le premier conduit (22, 222) d'un dispositif d'accès vasculaire (1) ;
- un dispositif de régulation et/ou d'interception du débit (104) prédisposé à réguler et/ou à interrompre le débit d'un liquide dans la canule médicale (103) ;
- un élément de connexion (106), fixé à la deuxième extrémité (109) de la canule médicale (108) pour permettre la connexion hydraulique à un tube d'entrée de sang ou à un tube de sortie de sang d'un appareil d'hémodialyse ;
- éventuellement, un élément d'accès disposé entre la première extrémité (108) de la canule et le dispositif de régulation et/ou d'interception (104) et définissant un canal d'accès (181) relié hydrauliquement à la première extrémité (108) de la canule ; et un moyen de fermeture réversible (180) pour fermer de manière réversible le canal d'accès (181) à une extrémité distale de l'élément d'accès afin de permettre l'injection d'un liquide dans le canal d'accès (181) et de provoquer l'écoulement du liquide vers la première extrémité (108) de la canule médicale (103) ; et
- éventuellement, un premier élément de préhension (120), placé entre la première extrémité (108) et/ou le dispositif de régulation et/ou d'interception (104), et/ou un second élément de préhension (130), placé à proximité de l'élément de connexion (106) ;
- dans lequel le système de collecte et/ou d'injection (1000) comprend optionnellement au moins un dispositif de cicatrisation (500) d'un accès vasculaire comprenant :
- une partie d'insertion relative (502) qui peut être insérée à l'intérieur d'un accès vasculaire et qui comprend : un cylindre (505) ayant un axe de développement longitudinal relatif : une première et une deuxième extrémités relatives qui sont longitudinales ; et une pointe distalement effilée (503) fixée à la première extrémité du cylindre (505) ; dans laquelle la partie d'insertion peut être insérée dans le deuxième conduit (33, 233) ou dans le troisième conduit (333) d'un dispositif d'accès vasculaire (1) pour hémodialyse selon l'une quelconque des revendications 5 à 9 à partir de la deuxième extrémité relative (32, 232, 332) de manière à occlure exclusivement une partie du deuxième conduit (33, 233) ou du troisième conduit (333) comprise entre, respectivement, la première valve (50) et la première plaque (4) ou entre la deuxième valve (50) et la deuxième plaque (304) ;
- une partie de connexion relative (504) fixée à la deuxième extrémité du cylindre (505) ; et
- une première et une seconde languette de préhension (501), fixées à la partie de connexion (504) sur les côtés opposés de celle-ci et sur les côtés opposés par rapport à l'axe du cylindre et avec une possibilité de mouvement relatif par rapport à la partie de connexion (504) afin de prendre : une première configuration relative dans laquelle la première et la deuxième languette de préhension (501) sont sensiblement coplanaires ; et une deuxième configuration dans laquelle les languettes sont opposées l'une à l'autre, dans laquelle, dans la première et la deuxième configuration, la première et la deuxième languette de préhension (501) ne peuvent pas être insérées dans le deuxième conduit (33, 233) ou le troisième conduit (333), où, optionnellement, le cylindre relatif est incliné par rapport à la partie de connexion d'un angle (λ) identique à l'angle (β) avec lequel le deuxième élément tubulaire (3, 203) ou le troisième élément tubulaire (303) est incliné par rapport, respectivement, à la première plaque (4) ou à la deuxième plaque (304).
